(19) Europäisches Patentamt
European Patent Office
Office européen des brevets

(11) Publication number: **0 095 584**
**B1**

(12) # EUROPEAN PATENT SPECIFICATION

(45) Date of publication of patent specification: **07.01.87**

(21) Application number: **83104221.3**

(22) Date of filing: **29.04.83**

(51) Int. Cl.$^4$: **C 07 D 403/12,**
C 07 D 417/12, C 07 K 5/06,
C 07 F 9/65, C 07 D 207/16,
A 61 K 31/505, A 61 K 31/53

(54) **Substituted 4-phenoxy and 4-phenylthio prolines.**

(30) Priority: **30.04.82 US 373570**

(43) Date of publication of application:
**07.12.83 Bulletin 83/49**

(45) Publication of the grant of the patent:
**07.01.87 Bulletin 87/02**

(84) Designated Contracting States:
**BE CH DE FR GB IT LI LU NL SE**

(56) References cited:
**EP-A-0 000 833**
**EP-A-0 036 572**
**EP-A-0 042 639**
**EP-A-0 053 902**
**FR-A-2 444 669**
**FR-A-2 446 281**
**FR-A-2 478 632**
**GB-A-2 061 933**
**GB-A-2 078 733**

**The file contains technical information submitted after the application was filed and not included in this specification**

(73) Proprietor: **E.R. Squibb & Sons, Inc.**
**Lawrenceville-Princeton Road**
**Princeton, N.J. 08540 (US)**

(72) Inventor: **Haugwitz, Rudiger D.**
**16 River Road**
**Titusville, NJ (US)**
Inventor: **Sprague, Peter W.**
**23 Timberlane Drive**
**Pennington, NJ (US)**

(74) Representative: **Vossius & Partner**
**Siebertstrasse 4 P.O. Box 86 07 67**
**D-8000 München 86 (DE)**

(56) References cited:
**US-A-4 168 267**
**US-A-4 316 906**

**BURGER'S MEDICINAL CHEMISTRY, 4th Edition, Part III, 165-170, 174, 175**

**EP 0 095 584 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

US—A—4,316,909, discloses ether and thioether mercaptoacyl prolines of the formula

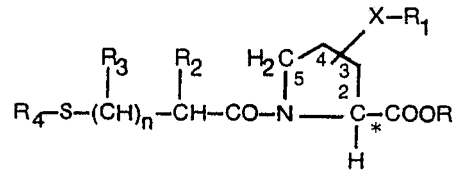

wherein

the group X—$R_1$ is located at the 3- or 4-position in the ring;

X is oxygen or sulfur;

R is hydrogen or lower alkyl;

$R_1$ is lower alkyl, lower alkenyl, lower alkynyl, cycloalkyl 1- or 2-adamantyl, aryl, substituted aryl, phenyl-lower alkylene or substituted phenyl-lower alkylene;

$R_2$ and $R_3$ are independently selected from hydrogen, lower alkyl, and trifluoromethyl;

$R_4$ is hydrogen. $R_5$—CO— or

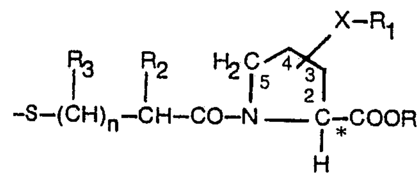

$R_5$ is lower alkyl, phenyl, phenyl-lower alkylene; substituted phenyl, or substituted phenyl-lower alkylene;

n is 0, 1 or 2; and salts thereof, which are useful as hypotensive agents.

The object of the present invention is to provide novel compounds having hypotensive and diuretic properties.

This object is achieved by the new substituted 4-phenoxy or 4-phenylthio prolines and salts thereof of the formula

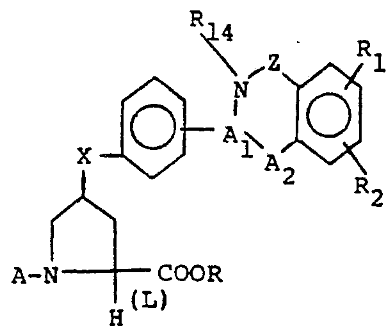

(I)

wherein

X is oxygen or sulfur.

$-A_1-A_2-$ is $-CH-NH-$ or $-C{=}N-$ .

A is 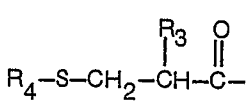 , 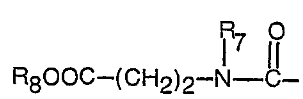 ,

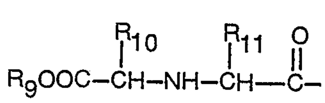 , or 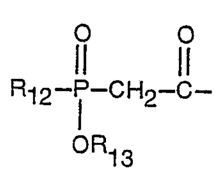 .

R, $R_8$ and $R_9$ are independently selected from hydrogen, $C_1$—$C_7$-alkyl, benzyl, benzhydryl, and salt forming ion.

$R_1$ and $R_2$ are independently selected from hydrogen, halogen, $C_1$—$C_7$-alkyl, $C_1$—$C_7$-alkoxy, halo substituted $C_1$—$C_7$-alkyl, nitro, and —$SO_2NH_2$.

Z is 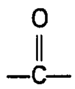 or 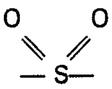

$R_3$ is hydrogen, $C_1$—$C_7$-alkyl, halo substituted $C_1$—$C_7$-alkyl, phenyl, benzyl, phenethyl, or $C_3$—$C_7$-cycloalkyl

$R_4$ is hydrogen or 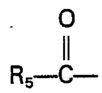

$R_5$ is $C_1$—$C_7$-alkyl

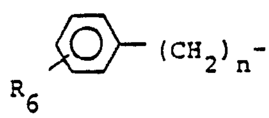 , 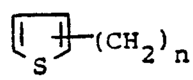 ,

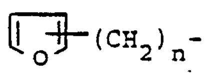 or 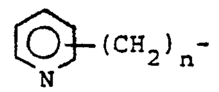 .

n is zero, one, two, three or four.

$R_6$ is hydrogen, $C_1$—$C_7$-alkyl, $C_1$—$C_7$-alkoxy, halogen, or hydroxy.

$R_7$ is $C_1$—$C_7$-alkyl or $C_3$—$C_7$-cycloalkyl.

$R_{10}$ is hydrogen, $C_1$—$C_7$-alkyl,

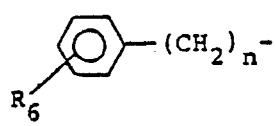 ,

halo substituted $C_1$—$C_7$-alkyl, hydroxy substituted $C_1$—$C_7$-alkyl, —$(CH_2)_q$ —$C_3$—$C_7$-cycloalkyl, —$(CH_2)_q$—N($C_1$—$C_7$-alkyl)$_2$, —$(CH_2)_q$—$NH_2$, —$(CH_2)_q$-carboxy, —$(CH_2)_q$—SH, —$(CH_2)_q$—S—$C_1$—$C_7$-alkyl,

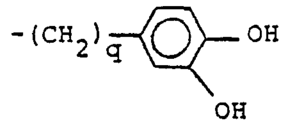 , 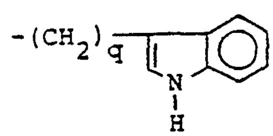 ,

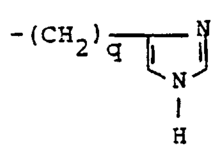 , $-(CH_2)_q$-guanidinyl,

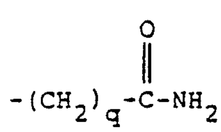 , 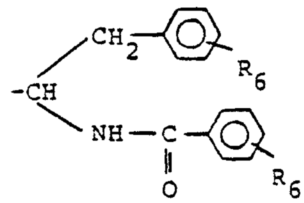 ;

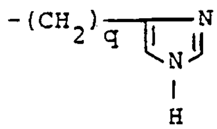 , $-(CH_2)_q$-guanidinyl,

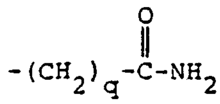 , or 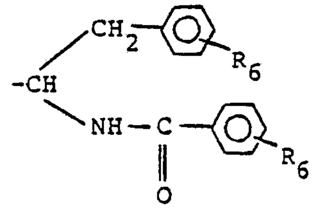 .

q is one, two, three or four.

$R_{11}$ is hydrogen, $C_1$—$C_7$-alkyl, halo substituted $C_1$—$C_7$-alkyl, hydroxy substituted $C_1$—$C_7$-alkyl, $—(CH_2)_q—NH_2$, $—(CH_2)_q—N(C_1—C_7\text{-alkyl})_2$,

$-(CH_2)_q$-guanidinyl, 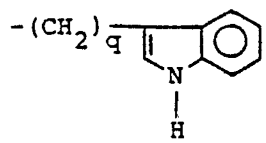 ,

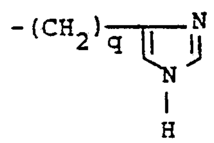 , 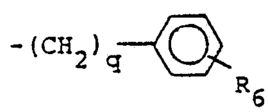 ,

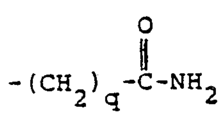 , $-CH_2-S-(CH_2)_2-NH_2$ ,

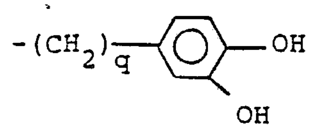 , 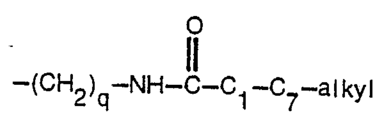 or

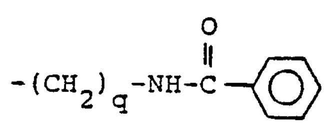 .

$R_{12}$ is $C_1$—$C_{10}$-alkyl,

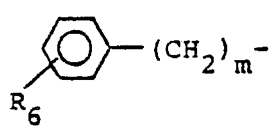 , 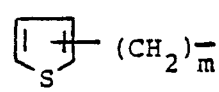 ,

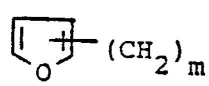 , 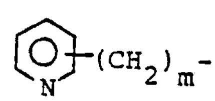 or

$C_3-C_7$-cycloalkyl$-(CH_2)_m-$ .

m is zero or an integer from 1 to 7.

$R_{13}$ is hydrogen, $C_1$—$C_7$-alkyl, benzyl, benzhydryl, salt forming ion or

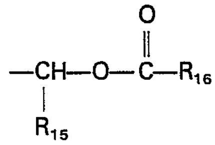

$R_{15}$ is hydrogen, $C_1$—$C_7$-alkyl, $C_3$—$C_7$-cycloalkyl or phenyl.
$R_{16}$ is hydrogen, $C_1$—$C_7$-alkyl, $C_1$—$C_7$-alkoxy, $C_3$—$C_7$-cycloalkyl, phenyl, benzyl, or phenethyl.
$R_{14}$ is hydrogen, $C_1$—$C_7$-alkyl, cycloalkyl—$(CH_2)_n$-,

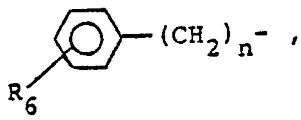 ,

halo substituted $C_1$—$C_7$-alkyl, hydroxy substituted $C_1$—$C_7$-alkyl, —$(CH_2)_q$—N($C_1$—$C_7$-alkyl$)_2$, or —$(CH_2)_q$—$NH_2$.

The symbols

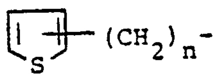 , 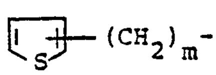 , 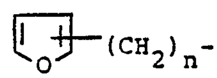 ,

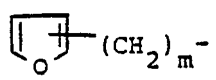 , 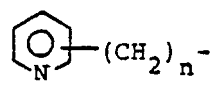 , and 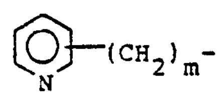

represent that the alkylene bridge is attached to an available carbon atom.

Examples for the alkyl residues as used in defining various symbols are straight or branched chain hydrocarbon radicals, for example, methyl, ethyl, propyl, isopropyl, butyl, t-butyl or pentyl. The preferred alkyl groups have up to four carbons with methyl and ethyl being most preferred. Similarly, the term alkoxy refers to such alkyl groups attached to an oxygen.

The term halogen refers to chloro, bromo, and fluoro. The term halo substituted alkyl refers to such alkyl groups described above in which one or more hydrogens have been replaced by chloro, bromo, or fluoro groups such as trichloroethyl, which is preferred, 2,2,2-trichloroethyl, chloromethyl, bromomethyl, etc. Similarly, the term hydroxy substituted alkyl refers to such alkyl groups in which one or more hydrogens have been replaced by hydroxy such as hydroxymethyl, 2-hydroxyethyl, etc.

Preferred cycloalkyl residues are the cyclopentyl and cyclohexyl group.

The compounds of formula I wherein A is

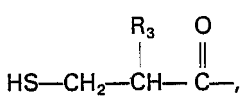

$A_1$—$A_2$ is —CH—NH—, and R is hydrogen are prepared by treating a compound of the formula

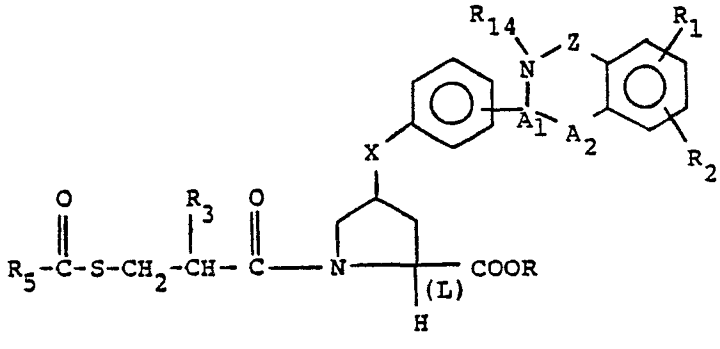

(II)

wherein R is a $C_1$—$C_7$-alkyl group such as methyl with base such as sodium hydroxide.

The compounds of formula II wherein $A_1$—$A_2$ is —CH—NH— are prepared by reacting a substituted phenoxy or phenylthio proline ester of the formula

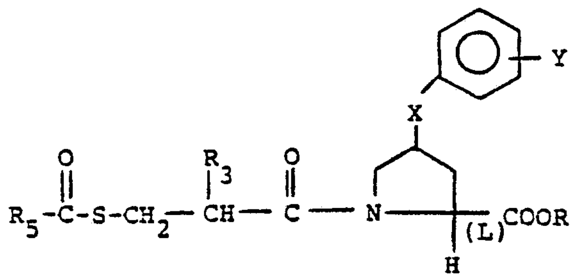

(III)

wherein Y is

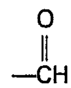

or —CH($C_1$—$C_7$-alkoxy)$_2$ with a substituted benzenamine of the formula

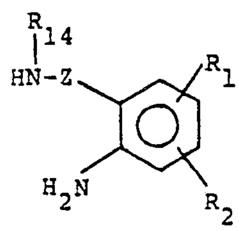

(IV)

The intermediates of formula III are obtained by treating the 4-hydroxy substituted proline esters of the formula

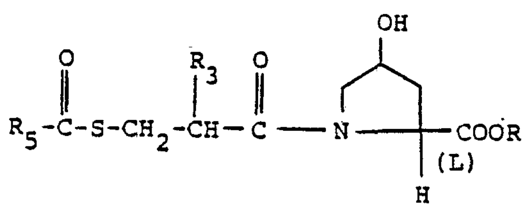

(V)

with the substituted phenol or thiophenol of the formula

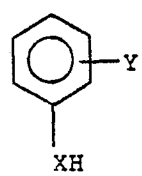

(VI)

in the presence of triphenylphosphine and dialkylazodicarboxylate.

Similarly, the compounds of formula I wherein A is

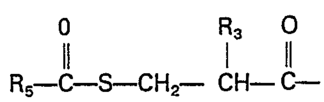

and R is hydrogen are prepared by treating a compound of formula II wherein R is an acid cleavable ester group such as benzhydryl with p-toluene-sulfonic acid and anisole.

The benzhydryl ester compounds of formula II are prepared analogous to the above procedure except

that the 4-hydroxy substituted proline acid of formula V (R is hydrogen) is treated with diphenyldiazomethane to yield the corresponding benzhydryl ester which is then reacted as described above.

The compounds of formula I wherein A is

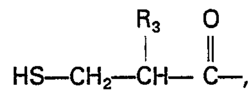

$A_1$—$A_2$ is —C=N—, and R is hydrogen are prepared by treating a compound of the formula

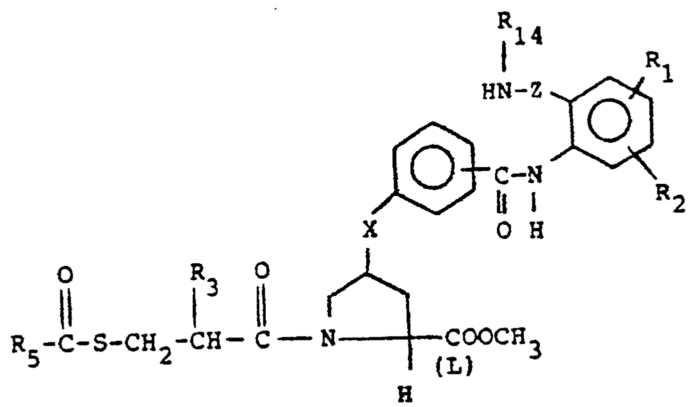

(VII)

with a concentrated aqueous solution of ammonia, i.e., ammonium hydroxide, followed by treatment with sodium hydroxide to remove the methyl ester group.

The intermediates of formula VII are prepared by reacting a substituted phenoxy or phenylthio proline ester of the formula

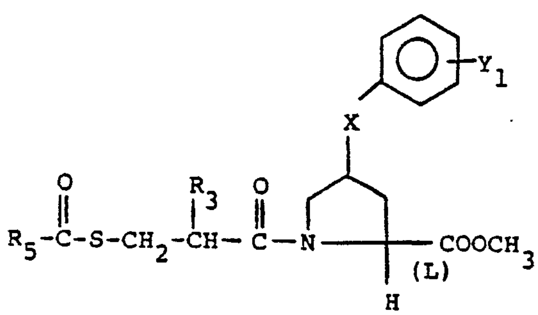

(VIII)

wherein $Y_1$ is

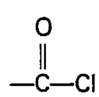 or 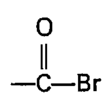

with a substituted benzeneamine of formula IV in a refluxing solvent such as dioxane. The compounds of the formula VIII are prepared by treating the corresponding compound wherein $Y_1$ is COOH with an agent such as thionyl chloride or phosphorus tribromide in a solvent such as dichloromethane and with a catalyst such as dimethylformamide.

The compounds wherein $Y_1$ is COOH are prepared by reacting the ester compound of the formula

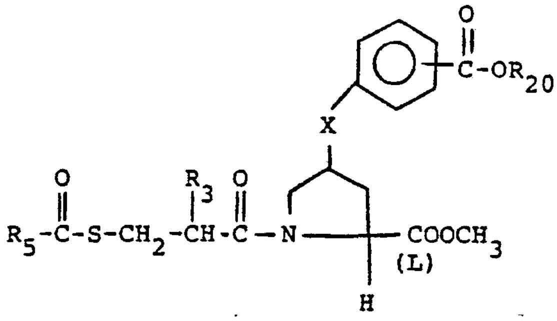

(IX)

wherein $R_{20}$ is an acid cleavable group such as t-butyl or benzhydryl, with an acid such as trifluoroacetic acid or 97% formic acid.

The ester compounds of formula IX are prepared by treating the 4-hydroxy substituted proline ester of formula V wherein R is methyl with the substituted phenol or thiophenol of the formula

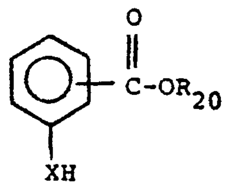

(X)

in the presence of triphenylphosphine and dialkylazodicarboxylate.

The compounds of formula I wherein A is

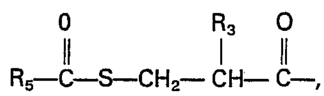

$A_1$—$A_2$ is —C=N—, and R is hydrogen are prepared by treating the corresponding compound wherein A is

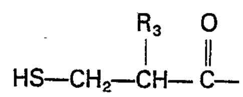

with an acid

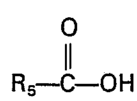

in the presence of a suitable activating agent such as dicyclohexylcarbodiimide or with an activated derivative such as

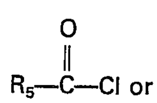 or 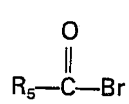

in the presence of a suitable acid acceptor such as pyridine or triethylamine.

The compounds of formula I wherein A is

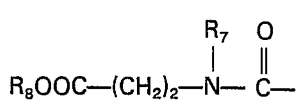

and $A_1$—$A_2$ is —CH—NH— are prepared by reacting a 4-hydroxy proline benzyl ester with an alkylamino-propanoate off the formula

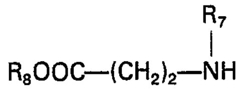

(XI)

wherein $R_8$ is lower alkyl in the presence of phosgene and N-methylmorpholine to yield the substituted 4-hydroxy proline benzyl ester of the formula

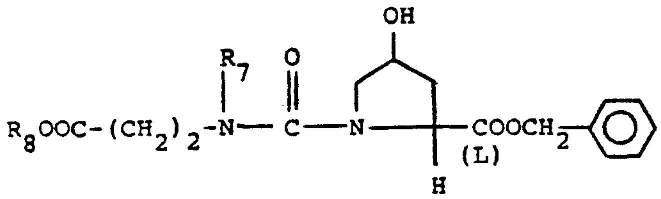

(XII)

Treatment of the product of formula XII with the substituted phenol or thiophenol of formula VI in the presence of triphenylphosphine and dialkylazodicarboxylate yields the dimethoxymethyl intermediate of the formula

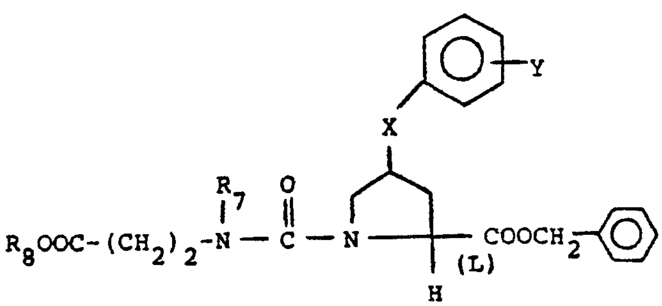

(XIII)

The intermediate of formula XIII is then reacted with the substituted benzenamine of formula IV to yield the proline benzyl ester

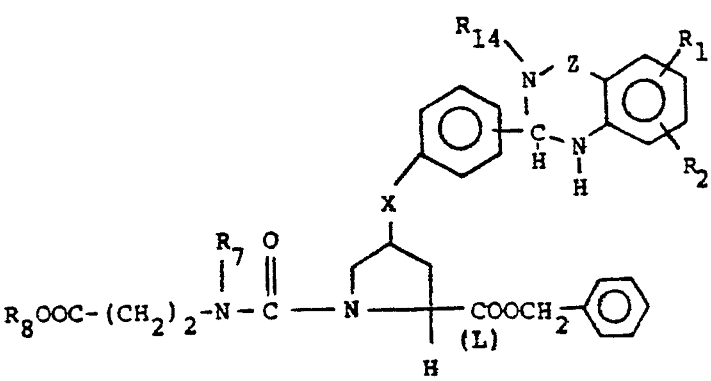

(XIV)

Hydrogenation of benzyl ester XIV with palladium/carbon removes the benzyl ester group and yields the products of formula I wherein $R_8$ is alkyl and R is hydrogen. Further treatment with aqueous sodium hydroxide yields the diacid product of formula I, i.e., both $R_8$ and R are hydrogen.

The compounds of formula I wherein A is

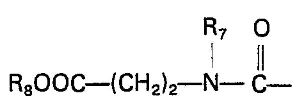

and $A_1$—$A_2$ is —C=N— are prepared by reacting the 4-hydroxyproline benzyl ester of formula XII with the substituted phenol or thiophenol of formula X in the presence of triphenylphosphine and dialkylazodicarboxylate to give the compound of the formula

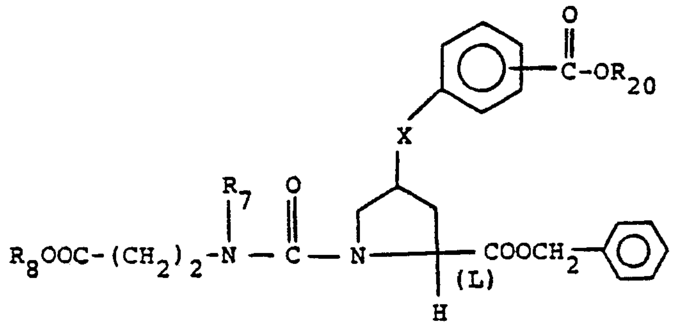

(XV)

wherein $R_8$ is $C_1$—$C_7$-alkyl and $R_{20}$ is an acid cleavable group such as t-butyl or benzhydryl.

Treatment of the compound of formula XV with trifluoroacetic acid or 97% formic acid gives the compound of the formula

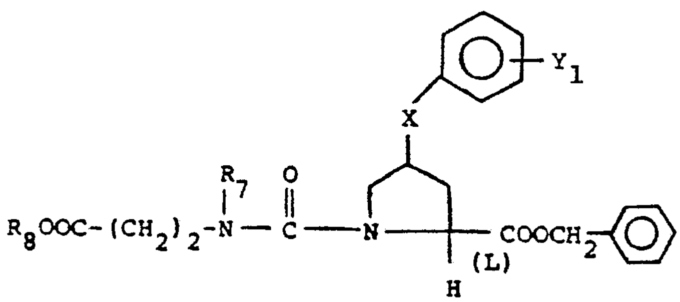

(XVI)

wherein $Y_1$ is —COOH. Treatment of the compound of formula XVI with an agent such as thionyl chloride or phosphorous tribromide as described above yield the compound wherein $Y_1$ is

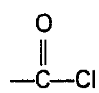 or 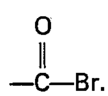

This intermediate is then reacted with the substituted benzeneamine of formula IV to give the compound of the formula

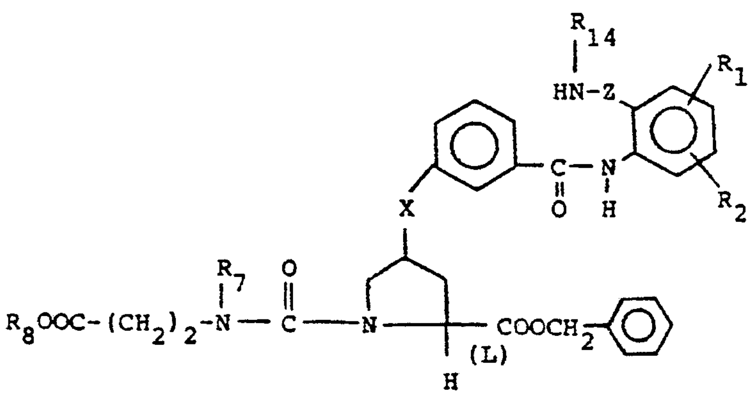

(XVII)

The compound of formula XVII is cyclized by treatment with a concentrated aqueous solution of ammonia to give the compound of the formula

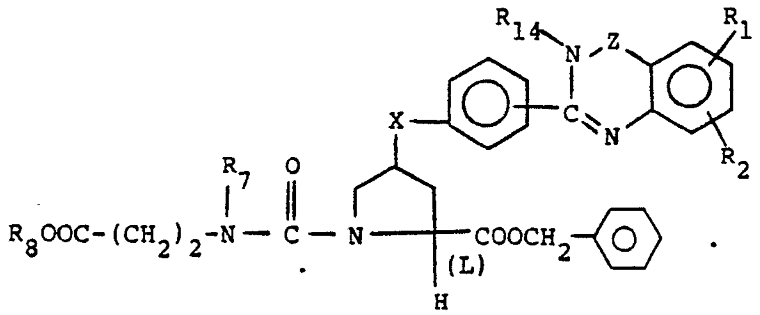

(XVIII)

Hydrogenation of benzyl ester XVIII with palladium/carbon removes the benzyl ester group and yields the products of formula I wherein $R_8$ is alkyl and R is hydrogen. Further treatment with aqueous sodium hydroxide yields the diacid product of formula I, i.e., both $R_8$ and R are hydrogen.

The compounds of formula I wherein A is

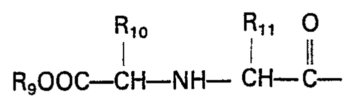

can be prepared by reacting an intermediate of the formula

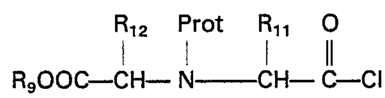

(XIX)

wherein Prot is a protecting group such as t-butoxycarbonyl with a functionalized proline ester of the formula

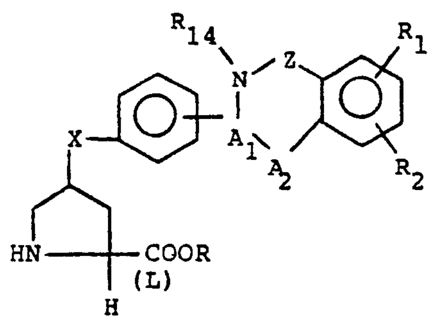

(XX)

in aqueous sodium bicarbonate to give the compound of the formula

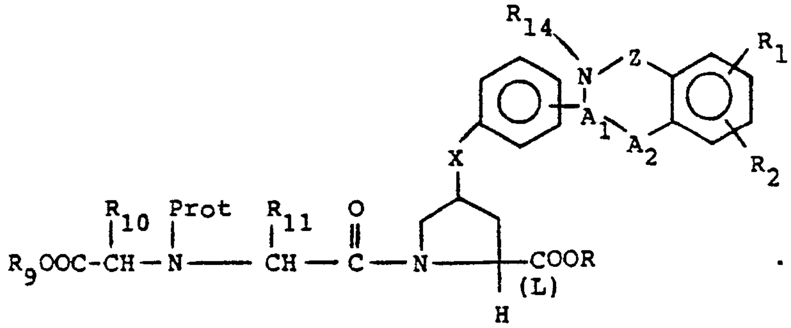

(XXI)

*Removal of the Prot group such as by treatment with trifluoroacetic acid gives the desired products of formula I.*

In these procedures when $R_9$ and R are carboxy ester protecting group such as $C_1$—$C_7$-alkyl or benzyl, they can be converted by known methods such as hydrolysis or hydrogenation to the products wherein R and/or $R_9$ are hydrogen. Reductive cleavage of the diester product wherein R is benzyl and $R_9$ is $C_1$—$C_7$-alkyl yields the monoester product where R is hydrogen and $R_9$ is $C_1$—$C_7$-alkyl. Similarly, reductive cleavage of the diester product wherein R is $C_1$—$C_7$-alkyl and $R_9$ is benzyl yields the monoester product wherein R is $C_1$—$C_7$-alkyl and $R_9$ is hydrogen.

The compounds of formula I wherein A is

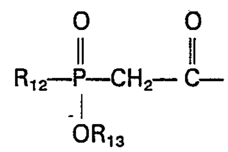

can be prepared by reacting a phosphinylacetic acid of the formula

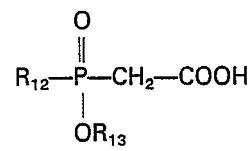

(XXII)

wherein $R_{13}$ is $C_1$—$C_7$-alkyl, benzyl or benzhydryl, with the functionalized proline ester of formula XX. The reaction can be accomplished using known amide bond forming procedures. For example, the reaction can be run in the presence of a coupling agent such as 1,1′-carbonyldiimidazole or the acid of formula XXII can be activated by formation of its mixed anhydride, symmetrical anhydride, acid halide, acid ester, etc.

The products of formula I wherein either or both of $R_{13}$ and R are $C_1$—$C_7$-alkyl, benzyl, or benzhydryl can be hydrogenated, for example, by treating with hydrogen in the presence of a palladium on carbon catalyst or chemically treated such as with sodium hydroxide in aqueous dioxane or with trimethylsilylbromide in dichloromethane to yield the products of formula I wherein $R_{13}$ and R are hydrogen.

The ester products of formula I wherein $R_{13}$ is

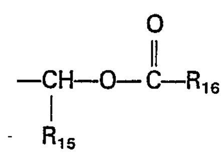

can be obtained by treating the product of formula I wherein $R_{13}$ is hydrogen or an alkali metal salt and R is benzyl or benzhydryl with the compound of the formula

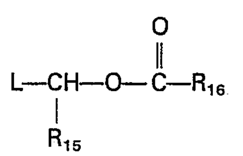

(XXIII)

wherein L is a leaving group such as chlorine, bromine, tolylsulfonyloxy, etc., in the presence of base. Removal of the R ester group such as by hydrogenation yields the products of formula I wherein $R_{13}$ is

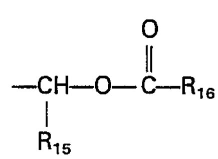

and R is hydrogen.

Of course, the product of formula I wherein A is

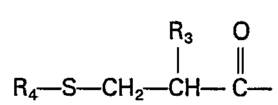 or 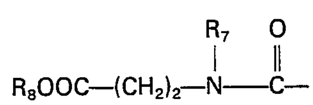

can also be prepared by coupling the appropriate sidechain with the functionalized proline ester of formula XX.

The starting materials of formula V are disclosed by Ondetti et al. in United States Patents 4,105,776 and 4,316,906. The alkylaminopropanoates of formula XI are described in the Tanabe patent applications noted above. The starting materials of formula XIX are described by Patchett et al. in European Patent Application 12,401 and United States Patent 4,374,829. The phosphinylacetic acid starting materials of formula XXII are described by Petrillo in United States Patents 4,168,267 and 4,337,201.

The substituted benzenamines of formula IV are described in the literature as note, for example, Cohen et al., JACS, Vol. 82, p. 273 (1960), Shetty et al., J. Med. Chem. Vol. 13, p. 886 (1970), and Close et al., JACS, Vol. 82, p. 1132.

The functionalized proline ester of formula XX wherein $A_1$—$A_2$ is —CH—NH— can be prepared by reacting an N-protected proline compound of the formula

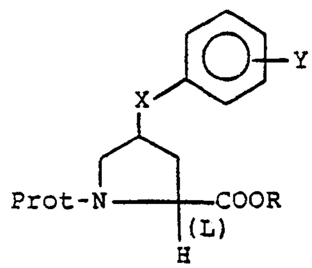

(XXIV)

wherein Prot is a protecting group such as t-butoxycarbonyl and Y is

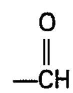

or —CH($C_1$—$C_7$-alkoxy)$_2$ with the substituted benzeneamine of formula IV in the presence of an acid such as toluenesulfonic acid and in a solvent such as acetonitrile.

The compound of formula XXIV can be prepared by reacting the 4-hydroxy-N-protected proline ester with the substituted phenol or thiophenol of formula VI in the presence of triphenylphosphine and dialkylazodicarboxylate.

The functionalized proline ester of formula XX wherein $A_1$—$A_2$ is —C=N— can be prepared by reacting an N-protected proline compound of the formula

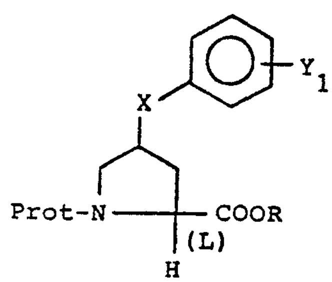

(XXV)

wherein Prot is a protecting group such as t-butoxycarbonyl and $Y_1$ is

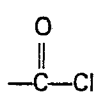 or 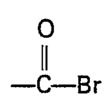

with the substituted benzeneamine of formula IV in a refluxing solvent such as dioxane.

The compounds of formula XXV are prepared from the corresponding compounds where $Y_1$ is —COOH by reacting with a reagent such as oxalyl chloride or phosphorus tribromide in the presence of the catalyst dimethylformamide in a solvent such as dichloromethane.

The corresponding carboxylic acid compounds of formula XXV can be prepared by treating the aldehyde, i.e., $Y_1$ is

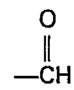

with an ozidizing agent such as chromium trioxide in the presence of a mixture of pyridine and dichloromethane.

In the above reactions if any or all of $R_{10}$, $R_{11}$ and $R_{14}$ are amino or hydroxy substituted $C_1$—$C_7$-alkyl,

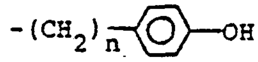 , 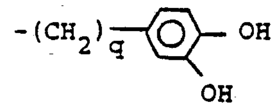 ,

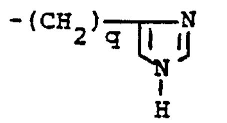 , $-(CH_2)_q-SH$,

or —$(CH_2)_q$-guanidinyl then the hydroxyl, amino, imidazolyl, mercaptan or guanidinyl function should be protected during the reaction. Suitable protecting groups include benzyloxycarbonyl, t-butoxycarbonyl, benzyl, benzhydryl and trityl, and nitro in the case of guanidinyl. The protecting group is removed by hydrogenation, treatment with acid, or other known means following completion of the reaction.

Preferred compounds of this invention are those of formula I wherein

X is oxygen,

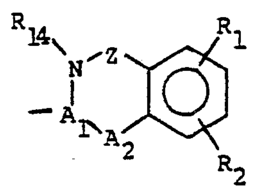 is 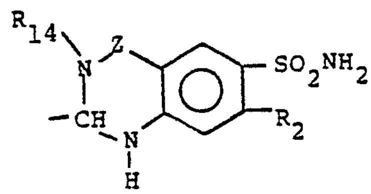 .

R is hydrogen, $C_1$—$C_4$-alkyl, or an alkali metal salt ion.

$R_{14}$ is hydrogen or $C_1$—$C_4$-alkyl, especially hydrogen or methyl.

$R_2$ is halogen, $C_1$—$C_4$-alkyl, or halo substituted $C_1$—$C_4$-alkyl, especially chloro or trifluoromethyl.

$R_4$ is hydrogen or

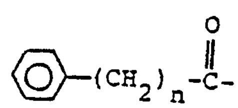

wherein n is zero, one, or two, especially hydrogen or

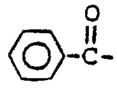 .

$R_3$ is $C_1$—$C_4$-alkyl, especially methyl.

$R_8$ is hydrogen, $C_1$—$C_4$-alkyl, or an alkali metal salt ion, especially hydrogen, ethyl, or an alkali metal salt ion.

$R_7$ is $C_1$—$C_4$-alkyl, especially ethyl.

$R_9$ is hydrogen, $C_1$—$C_4$-alkyl, or an alkali metal salt ion, especially hydrogen, ethyl, or an alkali metal salt ion.

$R_{10}$ is

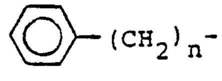

wherein n is zero, one, two, three or four, especially

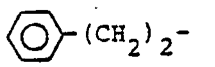 .

$R_{11}$ is $C_1$—$C_4$-alkyl, —$CH_2$—S—$(CH_2)_2$—$NH_2$, or —$(CH_2)_4$—$NH_2$, especially methyl.
$R_{12}$ is $C_1$—$C_{10}$-alkyl

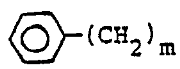

wherein m is zero or an integer from 1 to 7, especially

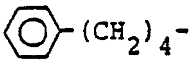 .

$R_{13}$ is hydrogen, alkali metal salt ion, or

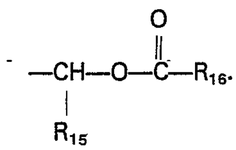

$R_{15}$ is hydrogen, straight or branched chain $C_1$—$C_4$-alkyl, or cyclohexyl.
$R_{16}$ is straight or branched chain $C_1$—$C_4$-alkyl.

The compounds of formula I give rise to cis-trans isomerism at the 4-position of the proline ring. The configuration of the final product depends upon the configuration of the 4-hydroxy proline starting material. When the hydroxy group is in the trans-configuration, the substituted phenol or phenylthiol intermediate is obtained in the cis-configuration and this configuration is maintained throughout the remainder of the reaction sequence. Similarly, if a cis-hydroxy starting material is employed the final product will be obtained in the trans configuration.

The compounds wherein A is

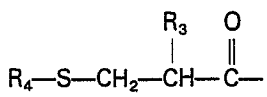 or 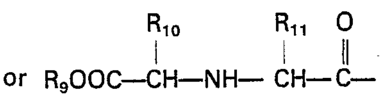

and $R_3$, $R_{10}$ and $R_{11}$ are other than hydrogen contain one or more additional asymmetric centers. These products of formula I can accordingly exist in stereoisomeric forms or as racemic mixtures thereof. The synthesis described above can utilize the racemate or one of the enantiomers as starting materials. When the racemic starting material is used in the synthetic procedure, the stereoisomers obtained in the final product can be separated by conventional chromatographic or fractional crystallization methods.

Preferably, if there is an asymmetric center in the sidechain it is in the S-configuration.

The compounds of this invention wherein at least one of R, $R_8$, $R_9$ and $R_{13}$ is hydrogen, form salts with various inorganic and organic bases which are also within the scope of the invention. Such salts include ammonium salts, alkali metal salts like lithium, sodium and potassium salts (which are preferred), alkaline earth metal salts like calcium and magnesium salts, salts with organic bases, e.g., dicyclohexylamine salt, benzathine, N-methyl-D-glucamine, salts with amino acids like arginine and lysine. The nontoxic, physiologically acceptable salts are preferred, although other salts are also useful, e.g., in isolating or purifying the product. The salts are formed using conventional techniques.

The compounds of formula I, and the physiologically acceptable salts thereof, are hypotensive agents. They inhibit the conversion of the decapeptide angiotensin I to angiotensin II and, therefore, are useful in reducing or relieving angiotensin related hypertension. The action of the enzyme renin on angiotensinogen, a pseudoglobulin in blood plasma, produces angiotensin I. Angiotensin I is converted by angiotensin converting enzyme (ACE) to angiotensin II. The latter is an active pressor substance which has been implicated as the causative agent in several forms of hypertension in various mammalian species, e.g., humans. The compounds of this invention intervene in the angiotensin → (renin) → angiotensin I → angiotensin II sequence by inhibiting angiotensin converting enzyme and reducing or eliminating the formation of the pressor substance angiotensin II. The compounds of formula I also possess diuretic activity. Thus, by the administration of a composition containing one or a combination of the compounds of the invention, hypertension in a species of mammal (e.g., humans) suffering therefrom is alleviated. A single dose, or preferably two to four divided daily doses, provided on a basis of about 0.1 to 100 mg. per kilogram of body weight per day is appropriate to reduce blood pressure. The substance is preferably

administered orally, but parenteral routes such as the subcutaneous, intramuscular, intravenous or intraperitoneal routes can also be employed.

The compounds of formula I can be formulated for use in the reduction of blood pressure in compositions such as tablets, capsules or elixirs for oral administration, or in sterile solutions or suspensions for parenteral administration. About 10 to 500 mg. of a compound of formula I is compounded with physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

The following examples are illustrative of the invention. Temperatures are given in degrees centigrade. AG-50W-X8 refers to a crosslinked polystyrene divinylbenzene sulfonic acid cation exchange resin. HP-20 refers to a porous crosslinked polystyrene-divinyl benzene polymer resin.

Example 1

[1(S),4S]-4-[3-[6-(Aminosulfonyl)-7-chloro-1,2,3,4-tetrahydro-4-oxo-2-quinazolinyl]phenoxy]-1-(3-mercapto-2-methyl-1-oxopropyl)-L-proline

a) [1(S),4R]-1-[3-(Benzoylthio)-2-methyl-1-oxopropyl]-4-hydroxy-L-proline, methyl ester

(D)-3-(Benzoylthio)-2-methylpropanoic acid (56.05 g., 0.25 mole) is suspended in 62.55 ml. of toluene. The temperature falls to 15° and then 0.386 ml. of dimethylformamide is added. Then 32.7 g. (0.25 mole + 10% excess) of thionyl chloride is added all at once with stirring. The temperature of the reaction mixture falls to 12° and the container used to measure the thionyl chloride is rinsed with 21.2 ml. of toluene which is then added to the reaction mixture. The temperature is raised gradually to 35° and maintained there for one hour. The reaction mixture is allowed to stir at room temperature overnight. The solvent and excess thionyl chloride are removed and the residue is treated twice with 100 ml. of toluene after which the toluene is removed to yield 63.4 g. of (D)-3-(benzoylthio)-2-methylpropanoic acid chloride.

L-4-Hydroxyproline (32.7 g., 0.25 mole) is dissolved in 250 ml. of water at pH 5.8. About 60 ml. of 10% sodium carbonate is added to bring the pH to 9.3. The solution is warmed to 30° and a toluene solution (75 ml.) containing 63 g. of (D)-3-(benzoylthio)-2-methylpropanoic acid chloride is added simultaneously with 10% aqueous sodium carbonate over one hour at 30° maintaining the pH at 9.0. The solution is stirred at pH 9.0 for 1.5 hours and the toluene layer is separated off. The aqueous layer is made strongly acid with concentrated HCl and the crystalline solid is filtered, washed with water, and air-dried to yield 71.4 g. of [1(S),4R]-1-[3-(benzoylthio)-2-methyl-1-oxopropyl]-4-hydroxy-L-proline; m.p. 195—196°. Recrystallization from alcohol gives a m.p. of 196—197°; $[\alpha]_D^{25}$ −139° (c = 1, methanol).

Anal. Calc'd. for $C_{16}H_{19}NO_5S$: N, 4.15; C, 56.95; H, 5.67; S, 9.50
Found: N, 3.96; C, 56.56; H, 5.77; S, 9.50.

A solution of 33.7 g. (0.1 mole) of [1(S),4R]-1-[3-(benzoylthio)-2-methyl-1-oxopropyl]-4-hydroxy-L-proline and 500 mg. of *p*-toluenesulfonic acid in 1 l. of methanol is gently refluxed for about 18 hours. The methanol is removed to yield 37 g. of a viscous residue which is dissolved in 1400 ml. of ether. The ether solution is washed twice with 250 ml. of water, twice with 250 ml. of 5% sodium bicarbonate, and once with 250 ml. of brine and dried over $MgSO_4$. The ether is removed to yield 27.3 g. of crystalline product (after trituration with petroleum ether); m.p. 64—65°. Recrystallization from ether yields [1(S),4R]-1-[3-(benzoylthio)-2-methyl-1-oxopropyl]-4-hydroxy-L-proline, methyl ester; m.p. 65.5—67°; $[\alpha]_D^{25}$ −158° (c = 1, methanol).

Anal. calc'd. for $C_{17}H_{21}NO_5S$: N, 3.99; C, 58.10; H, 6.02; S, 9.12
Found: N, 3,96; C, 57.96; H, 6.09; S, 9.11.

b) 3-(Dimethoxymethyl)phenol

To a mixture of 12.2 g. (0.1 mole) of 3-hydroxybenzaldehyde and 11.0 ml. of trimethyl orthoformate in 8 ml. of methanol there is added 1 drop of concentrated hydrochloric acid. The reaction mixture becomes warm. The reaction mixture is heated to 55° for 1.5 hours, then cooled and the solvent is removed *in vacuo*. The dark colored residue is dissolved in ethyl ether, filtered, and evaporated *in vacuo* to give 6.5 g. of greenish liquid 3-(dimethoxymethyl)phenol. Tlc (ethyl ether) $R_f$ = 0.8 (single spot).

c) [1(S),4S]-1-[3-(Benzoylthio)-2-methyl-1-oxopropyl]-4-[3-dimethoxymethyl)phenoxy]-L-proline, methyl ester

To a solution of 4.2 g. (0.012 mole) of [1(S),4R]-1-[3-(benzoylthio)-2-methyl-1-oxopropyl]-4-hydroxy-L-proline, methyl ester, 3 g. (0.0178 mole) of 3-(dimethoxymethyl) phenol and 4.6 g. (0.0175 mole) of triphenylphosphine in 15 ml. of dry tetrahydrofuran there is added 3.1 g. of diethylazodicarboxylate in 5 ml. of dry tetrahydrofuran (reaction mixture becomes warm) and the mixture is stirred overnight at room temperature under argon. The solvent is removed *in vacuo* and the resulting oil is dissolved in 20 ml. of 1:1 petroleum ether/ethyl ether. The mixture is seeded with dicarbethoxyhydrazine and allowed to stand at room temperature for 1 hour. The resulting solid is filtered off and washed with ethyl ether. The filtrate is preabsorbed on Baker silica gel and flash chromatographed. Elution with 4 l. of petroleum ether/ethyl ether (1:1) followed by 1 l. of petroleum ether/ethyl ether (1:9) yields 0.9 g. of pure [1(S),4S]-1-[3-(benzoylthio)-2-methyl-1-oxopropyl]-4-[3-(dimethoxymethyl)phenoxy]-L-proline, methyl ester as a colorless viscous oil. Tlc (ethyl ether) $R_f$ = 0.4.

d) 2-Amino-4-chloro-5-sulfamylbenzamide

A solution of 14 g. of 4-chloro-5-sulfamyl-N-acetylanthranilic acid and 84 ml. of 3N sodium hydroxide is refluxed for 3 hours, filtered, and the filtrate is adjusted to pH 6. The flocculant material is filtered off and the filtrate is adjusted to pH 3. The resulting solid is filtered off and dried to yield 10.8 g. of 4-chloro-5-sulfamylanthranilic acid; m.p. 276—277° (dec.).

A solution of 10 g. of 4-chloro-5-sulfamylanthranilic acid, 100 ml. of methanol and 5 ml. of concentrated sulfuric acid is refluxed for 24 hours, cooled and poured into 700 ml. of concentrated ammonia and stirred at room temperature for 7 days during which time the solution becomes clear. The solution is partially evaporated *in vacuo* until a white solid formed. The solid is filtered off to yield 5.3 g. of 2-amino-4-chloro-5-sulfamyl-benzamide; m.p. 278—280°.

e) [1(S),4S]-4-[3-[6-(Aminosulfonyl)-7-chloro-1,2,3,4-tetrahydro-4-oxo-2-quinazolinyl]phenoxy]-1-[3-(benzoylthio)-2-methyl-1-oxopropyl]-L-proline, methyl ester

A mixture of 0.5 g. (0.001 mole) of [1(S),4S]-1-[3-(benzoylthio)-2-methyl-1-oxopropyl]-4-[3-(dimethoxymethyl)phenoxy]-L-proline, methyl ester and 0.25 g. of 2-amino-4-chloro-5-sulfamylbenzamide in 10 ml. of acetonitrile is treated with 0.001 g. of p-toluenesulfonic acid (transient yellow color) and the mixture is refluxed for 1 hour. The solvent is removed *in vacuo* and the residue is triturated with ethyl ether to yield a solid which is dissolved in dimethyl sulfoxide. The dimethyl sulfoxide solution is diluted with water and the resulting solid is filtered off and dried overnight at 80° to yield 0.5 g. of [1(S),4S]-4-[3-[6-(aminosulfonyl)-7-chloro-1,2,3,4-tetrahydro-4-oxo-2-quinazolinyl]phenoxy]-1-[3-(benzoylthio)-2-methyl-1-oxopropyl]-L-proline, methyl ester; m.p. 125° (dec.). Tlc (ethyl acetate) $R_f$ = 0.3 visualized with vanillin; (dichloromethane/methanol/acetic acid; 90:5:5) $R_f$ = 0.8 visualized with PMA.

| | | | | | |
|---|---|---|---|---|---|
| Anal. Calc'd. for $C_{31}H_{31}ClN_4O_8S_2 \cdot 0.5H_2O$: | C, 53.56; | H, 4.49; | N, 8.06; | Cl, 5.10; | S, 9.22 |
| Found: | C, 53.64; | H, 4.50; | N, 7.99; | Cl, 4.75; | S, 9.18. |

f) [1(S),4S]-4-[3-[6-(Aminosulfonyl)-7-chloro-1,2,3,4-tetrahydro-4-oxo-2-quinazolinyl]phenoxy]-1-(3-mercapto-2-methyl-1-oxopropyl)-L-proline

A mixture of 1 g. of [1(S),4S]-4-[3-[6-(aminosulfonyl)-7-chloro-1,2,3,4-tetrahydro-4-oxo-2-quinazolinyl]-phenoxy]-1-[3-(benzoylthio)-2-methyl-1-oxopropyl]-L-proline, methyl ester, 20 ml. of methanol, and 80 ml. of 0.2 N sodium hydroxide is stirred at room temperature for 8 hours. After this time, Tlc indicated the complete absence of starting material and mono ester. The reaction mixture is then acidified with 5% potassium bisulfate. The resulting solid is collected by filtration, washed with distilled water, and then dried *in vacuo* to yield 700 mg. of crude product.

The crude reaction product is dissolved in 15 ml. of methanol and chromatographed on 50 g. of Sephadex (LH — 20 column), by gravity, eluting with methanol to yield 630 mg. of [1(S),4S]-4-[3-[6-(aminosulfonyl)-7-chloro-1,2,3,4,-tetrahydro-4-oxo-2-quinazolinyl]phenoxy]-1-(3-mercapto-2-methyl-1-oxopropyl)-L-proline; m.p. 180—190°. Tlc (silica gel; dichloromethane (8)/methanol (1)/acetic acid (1)) $R_f$ = 0.5.

| | | | | | | |
|---|---|---|---|---|---|---|
| Anal. Calc'd. for $C_{23}H_{25}ClN_4O_7S_2 \cdot 0.5\ H_2O$: | C, 47.79; | H, 4.53; | N, 9.69; | Cl, 6.13; | S, 11.07; | SH, 5.77 |
| Found: | C, 47.80; | H, 4.66; | N, 9.47; | Cl, 6.08; | S, 10.91; | SH, 5.77. |

## Example 2

[1(S),4S]-4-[2-[6-Aminosulfonyl)-7-chloro-1,2,3,4-tetrahydro-4-oxo-2-quinazolinyl]phenoxy]-1-(3-mercapto-2-methyl-1-oxopropyl)-L-proline

a) 2-(Dimethoxymethyl)phenol

To a mixture of 24.4 g. of salicylaldehyde, 25 g. of trimethyl orthoformate, and 50 g. of methanol there is added 0.1 ml. of concentrated hydrochloric acid. The temperature rises at once and the solution is kept at 55° for 1 hour. Then the reaction mixture is concentrated *in vacuo* and the remaining oil is fractionated at 85—90°/0.05 mm. to yield 15 g. of 2-(dimethoxymethyl)phenol as a viscous, colorless oil.

b) [1(S),4S]-1-[3-(Benzoylthio)-2-methyl-1-oxopropyl]-4-[2-(dimethoxymethyl)phenoxy]-L-proline, methyl ester

To a mixture of 4.2 g. of [1(S),4R]-1-[3-(benzoylthio)-2-methyl-1-oxopropyl]-4-hydroxy-L-proline, methyl ester, 3 g. of 2-(dimethoxymethyl)phenol, 4.6 g. of triphenylphosphine dissolved in 15 ml. of tetrahydrofuran there is added 3.1 g. of diethylazodicarboxylate dissolved in 5 ml. of tetrahydrofuran (nitrogen atmosphere). The mixture becomes warm. The solution is stirred overnight. Addition of ether/petroleum ether yields 1.1 g. of dicarbethoxyhydrazine which is filtered off. Addition of more petroleum ether to the filtrate furnishes oily triphenylphosphine oxide which on trituration with ether turns solid; 2.5 g. The filtrate is absorbed on Baker silica and flash chromatographed. Elution with 85% ether — 10% petroleum ether elutes the pure product in six fractions, 250 ml. each, yielding 2.99 g. of [1(S),4S]-1-[3-(benzoylthio)-2-methyl-1-oxopropyl]-4-[2-(dimethoxymethyl)phenoxy]-L-proline, methyl ester. A small sample is characterized and analyzed as its 2,4-dinitrophenyl hydrazine derivative.

| | | | | |
|---|---|---|---|---|
| Anal. Calc'd. for $C_{30}H_{29}N_5O_9S$: | C, 56.68; | H, 4.60; | N, 11.02; | S, 5.04 |
| Found: | C, 56.43; | H, 4.58; | N, 10.99; | S, 5.04. |

c) [1(S),4S]-4-[2-[6-(Aminosulfonyl)-7-chloro-1,2,3,4-tetrahydro-4-oxo-2-quinazolinyl]phenoxy]-1-[3-(benzoylthio)-2-methyl-1-oxopropyl)-L-proline, methyl ester.

A mixture of 0.25 g. of 2-amino-4-chloro-5-sulfamylbenzamide, 0.5 g. of [1(S),4S]-1-[3-(benzoylthio)-2-methyl-1-oxopropyl]-4-[2-(dimethoxymethyl)phenoxy]-L-proline, methyl ester, and 0.1 g. of p-toluenesulfonic acid in 20 ml. of acetonitrile is refluxed overnight. About 10 ml. of solvent is removed *in vacuo* and the resulting solution is treated with enough ether to give complete precipitation of the product. The solid is filtered off and reprecipitated from dimethylformamide-water to yield 0.5 g. of [1(S),4S]-4-[2-[6-(aminosulfonyl)-7-chloro-1,2,3,4-tetrahydro-4-oxo-2-quinazolinyl]phenoxy]-1-[3-(benzoylthio)-2-methyl-1-oxopropyl]-L-proline, methyl ester. Tlc (dichloromethane/methanol/acetic acid; 90:5:5) $R_f$ = 0.61 visualized with PMA.

| | | | | | |
|---|---|---|---|---|---|
| Anal. Calc'd. for $C_{31}H_{31}ClN_4O_8S_2$: | C, 53.47; | H, 4.49; | N, 8.05; | Cl, 5.09; | S, 9.21 |
| Found: | C, 53.25; | H, 4.49; | N, 8.07; | Cl, 4.87; | S, 8.97. |

d) [1(S),4S]-4-[2-[6-(Aminosulfonyl)-7-chloro-1,2,3,4-tetrahydro-4-oxo-2-quinazolinyl]phenoxy]-1-(3-mercapto-2-methyl-1-oxopropyl)-L-proline

A mixture of 0.52 g. of [1(S),4S]-4-[2-[6-(aminosulfonyl)-7-chloro-1,2,3,4-tetrahydro-4-oxo-2-quinazolinyl]phenoxy]-1-[3-(benzoylthio)-2-methyl-1-oxopropyl]-L-proline, methyl ester, 10 ml. of methanol, and 2 ml. of 2N sodium hydroxide is stirred at room temperature for 1 hour (nitrogen atmosphere). At this time, no starting material is detectable by Tlc but a certain quantity of mono ester is still present. Thus, an additional 2 ml. of 2N sodium hydroxide is added and after two additional hours of stirring the reaction mixture is acidified with 10% potassium bisulfate. The resulting white precipitate is filtered off, washed with water and dried yielding 0.37 g. of [1(S),4S]-4-[2-[6-(aminosulfonyl)-7-chloro-1,2,3,4-tetrahydro-4-oxo-2-quinazolinyl]phenoxy]-1-(3-mercapto-2-methyl-1-oxopropyl)-L-proline; m.p. 190—195° (softening at 150°). Tlc (dichloromethane/methanol/acetic acid; 90:5:5) $R_f$ = 0.21 visualized with PMA.

| | | | | | |
|---|---|---|---|---|---|
| Anal. Calc'd. for $C_{23}H_{25}ClN_4O_7S_2 \cdot 0.8\ H_2O$: | C, 47.34; | H, 4.59; | N, 9.60; | Cl, 6.08; | S, 10.99 |
| Found: | C, 47.34; | H, 4.45; | N, 9.95; | Cl, 5.98; | S, 10.73. |

## Example 3

[1(S),4S]-4-[4-[6-(Aminosulfonyl)-7-chloro-1,2,3,4-tetrahydro-4-oxo-2-quinazolinyl]phenoxy]-1-(3-mercapto-2-methyl-1-oxopropyl)-L-proline

a) 4-(Dimethoxymethyl)phenol

A solution of 5 g. (0.04 mole) of 4-hydroxybenzaldehyde in 25 ml. of methanol is treated with a drop of concentrated hydrochloric acid and stored at 25° overnight. Then several scoops each of sodium bicarbonate and sodium sulfate are added and swirled for 10 minutes. The slurry is filtered, evaporated *in vacuo*, and the residue taken up in ether, filtered and evaporated. Evaporation with ethyl acetate and finally hexane gives 4.5 g. of solid 4-(dimethoxymethyl)phenol.

b) [1(S),4S]-1-[3-(Benzoylthio)-2-methyl-1-oxopropyl]-4-[4-(dimethoxymethyl)phenoxy]-L-proline, methyl ester

A solution of 6.18 g. (0.018 mole) [1(S),4R]-1-[3-(benzoylthio)-2-methyl-1-oxypropyl]-4-hdyroxy-L-proline, methyl ester, 4.61 g. (0.018 mole) of triphenylphosphine and 2.96 g. of 4-(dimethoxymethyl)phenol in 75 ml. of dry tetrahydrofuran at 0° under argon is treated with a solution of 3.07 g. (0.018 mole) of diethylazodicarboxylate in 5 ml. of tetrahydrofuran, and the mixture is allowed to come to 25° overnight. Tlc on silica (ethyl ether elution) shows considerable starting material as well as product. Therefore, another 2.96 g. (0.018 mole) of 4-(dimethoxymethyl)phenol is added (mixture turns green), followed by 4.6 g. (0.018 mole) of triphenylphosphine (still green) and 3.07 g. (0.018 mole) of diethylazodicarboxylate (back to yellow). After 9 hours at 25° virtually all of the starting material has disappeared (by Tlc). Two chromatographic purifications on 60—200 mesh silica in ether gives 4 g. of material which have a single spot by Tlc with ether, but two closely running spots in hexane/acetone (2:1). These are completely separated on the Waters 500 preparative LC on two silica columns in hexane/acetone (4:1); the mixture being injected in benzene. The less polar material (1.7 g.) is determined by proton and carbon NMR to be [1(S),4S]-1-[3-(benzoylthio)-2-methyl-1-oxopropyl]-4-[4-(dimethoxymethyl)phenoxyl]-L-proline, methyl ester.

c) [1(S),4S]-4-[4-[6-(Aminosulfonyl)-7-chloro-1,2,3,4-tetrahydro-4-oxo-2-quinazolinyl]phenoxy]-1-[3-(benozylthio)-2-methyl-1-oxopropyl]-L-proline, methyl ester

A slurry of 1.2 g (2.4 mmole) of [1(S),4S]-1-[3-(benzoylthio)-2-methyl-1-oxopropyl]-4-[4-(dimethoxymethyl)phenoxy]-L-proline, methyl ester and 0.6 g. (2.4 mmole) of 2-amino-4-chloro-5-sulfamylbenzamide in 50 ml. of dry acetonitrile is treated with 25 mg. of p-toluenesulfonic acid and refluxed for 3 hours. Tlc (silica, ethyl acetate) shows one major spot ($R_f$ = 0.2). The mixture is evaporated and triturated with isopropyl ether to give [1(S),4S]-4-[4-[6-(aminosulfonyl)-7-chloro-1,2,3,4-tetrahydro-4-oxo-2-quinazolinyl]phenoxy]-1-[3-(benzoylthio)-2-methyl-1-oxopropyl]-L-propline, methyl ester as a crude solid.

d) [1(S),4S]-4-[4-[6-(Aminosulfonyl)-7-chloro-1,2,3,4-tetrahydro-4-oxo-2-quinazolinyl]phenoxy]-1-(3-mercapto-2-methyl-1-oxopropyl)-L-proline

The entire sample of the crude product from part (c) is dissolved in 100 ml. of argon purged 0.2 N sodium hydroxide and kept under argon for 1 hour, pH = 14. Acidification (10% potassium bisulfate) and extraction with ethyl acetate, drying ($Na_2SO_4$), and evaporation gives 1.1 g. of white solid which shows 6 spots on Tlc [silica:dichloromethane(8)/methanol(1)/acetic acid(1)] two of which ($R_f$ = 0.37, 0.7) fluoresce bright blue under short wave UV irradiation. Chromatography on 200 g. of Sephadex (LH 20) in methanol gives the two fluoresing materials (as a mixture) as the first material off the column. Evaporation gives 0.8 g. of a foam. This material is dissolved in methanol-ethyl acetate and shaken with aqueous bicarbonate. The less polar material stayed in the organic layer. Acidification of the aqueous layer (10% potassium bisulfate) and extraction with ethyl acetate-methanol affords after drying ($Na_2SO_4$) and evaporation, the pure more polar product. This compound is dissolved in methanol, the solution filtered (millipore) and evaporated, and the resulting glass triturated with ether to give 0.5 g. of white solid [1(S),4S]-4-[4-[6-(aminosulfonyl)-7-chloro-1,2,3,4-tetrahydro-4-oxo-2-quinazolinyl]phenoxy]-1-(3-mercapto-2-methyl-1-oxopropyl-L-proline; m.p. 190—210°.

| | | | | | | |
|---|---|---|---|---|---|---|
| Anal. Calc'd. for $C_{23}H_{25}ClN_4O_7S_2$: | C, 48.54; | H, 4.43; | N, 9.85; | S, 11.27; | Cl, 6.23; | SH, 5.81 |
| Found: | C, 48.64; | H, 4.78; | N, 9.49; | S, 11.19; | Cl, 6.51; | SH, 5.67. |

## Example 4

[1(S),4S]-4-[4-[6-(Aminosulfonyl)-7-chloro-1,2,3,4-tetrahydro-4-oxo-2-quinazolinyl]phenoxy]-1-[3-(benzoylthio)-2-methyl-1-oxopropyl]-L-proline

a) [1(S),4R]-1-[3-(Benzoylthio)-2-methyl-1-oxopropyl]-4-hydroxy-L-proline, diphenylmethyl ester

An ether solution of diphenyldiazomethane is added to a slurry of [1(S),4R]-1-[3-(benzoylthio)-2-methyl-1-oxopropyl]-4-hydroxy-L-proline (5 g./100 ml. of dichloromethane). The reaction mixture is stirred at room temperature overnight. The reaction solution is then evaporated to residue. The residue is taken up in dichloromethane and washed with 1N sodium bicarbonate and brine. The solution is then dried ($Na_2SO_4$) and evaporated to a residue which is triturated with ether and recrystallized from ethyl acetate to give 6 g. of pure [1(S),4R]-1-[3-(benzoylthio)-2-methyl-1-oxopropyl]-4-hydroxy-L-proline, diphenylmethyl ester as a white crystalline solid.

b) [1(S),4S]-1-[3-(Benzoylthio)-2-methyl-1-oxopropyl]-4-[4-(dimethoxymethyl)phenoxy]-L-proline, diphenylmethyl ester

To a stirred mixture of 5.04 g (10 mmole) of [1(S),4R]-1-[3-(benzoylthio)-2-methyl-1-oxopropyl]-4-hydroxy-L-proline, diphenylmethyl ester, 2.52 g. (15 mmole) of 4-(dimethoxymethyl)phenol, and 3.93 g. (15 mmole) of triphenylphosphine in 15 ml. of dry tetrahydrofuran under argon at 0° is added dropwise a solution of diethylazodicarboxylate (2.61 g., 15 mmole) in 5 ml. of tetrahydrofuran. The reaction mixture is stirred at ambient temperature for approximately 64 hours and then concentrated to a residue. The residue is chromatographed on a gravity silica gel column (diethyl ether eluant) followed by Waters Prep. layer chromatography using hexane:acetone (4:1) to give [1(S),4S]-1-[3-(benzoylthio)-2-methyl-1-oxopropyl]-4-[4-(dimethoxymethyl)phenoxy]-L-proline, diphenylmethyl ester.

c) [1(S),4S]-4-[4-[6-(Aminosulfonyl)-7-chloro-1,2,3,4-tetrahydro-4-oxo-2-quinazolinyl]phenoxy]-1-[3-(benzoylthio)-2-methyl-1-oxopropyl]-L-proline

1 g. (1.5 mmole) of [1(S),4S]-1-[3-(benzoylthio)-2-methyl-1-oxopropyl]-4-[4-(dimethoxymethyl)phenoxy]-L-proline, diphenylmethyl ester, 0.5 g. (2 mmole) of 2-amino-4-chloro-5-sulfamoylbenzamide, and anisole (2 ml.) are combined in approximately 20 ml. of acetonitrile. A catalytic amount (50 mg.) of p-toluenesulfonic acid is added and the reaction mixture is heated at reflux for 1 hour. After evaporation of the solvent, the residue is taken up in ethyl acetate and water. The aqueous layer is discarded. The product is extracted into a sodium bicarbonate solution, washed with ethyl acetate and reacidified using potassium bisulfate. Upon acidification the product is extracted into fresh ethyl acetate. After washing with water and brine and drying ($MgSO_4$), the solvent is evaporated to give a white solid. Purification is done on a Sephadex LH20 column (methanol). Trituration with ether gives [1(S),4S]-4-[4-[6-(aminosulfonyl)-7-chloro-1,2,3,4-tetrahydro-4-oxo-2-quinazolinyl]phenoxy]-1-[3-(benzoylthio)-2-methyl-1-oxopropyl]-L-proline as a white crystalline solid.

| | | | | | |
|---|---|---|---|---|---|
| Anal. Calc'd. for $C_{30}H_{29}ClN_4O_8S_2 \cdot 0.25\ H_2O$: | C, 53.17; | H, 4.39; | N, 8.27; | S, 9.46; | Cl, 5.23 |
| Found: | C, 53.15; | H, 4.47; | N, 8.08; | S, 9.17; | Cl, 5.17. |

## Example 5

[1(S),4S]-4-[4-[7-(Aminosulfonyl)-6-chloro-3,4-dihydro-1,1-dioxo-2H-1,2,4-benzothiadiazin-3-yl]phenoxy]-1-(3-mercapto-2-methyl-1-oxopropyl)-L-proline

a) [1(S),4S]-4-[4-[7-(Aminosulfonyl)-6-chloro-3,4-dihydro-1,1-dioxo-2H-1,2,4-benzothiadiazin-3-yl]phenoxy]-1-[3-(benzoylthio)-2-methyl-1-oxopropyl]-L-proline, methyl ester

A slurry of 450 mg. (0.9 mmole) of [1(S),4S]-1-[3-(benzoylthio)-2-methyl-1-oxopropyl]-4-[4-(dimethoxymethyl)phenoxy]-L-proline, methyl ester and 280 mg. (1.0 mmole) of 4-amino-6-chloro-1,3-benzenedisulfonamide in 10 ml. of acetonitrile is treated with 20 mg. of p-toluenesulfonic and the resulting mixture

was heated to reflux for 13 hours. The solvent is evaporated *in vacuo* and the residue taken up in ethyl acetate and passed through a 40 ml. silica (60—200 mesh) pad to remove some polar (Tlc on silica, hexane/acetone 2:1) impurities. Trituration of the residue from the evaporation of the ethyl acetate with dichloromethane removes the less polar impurities ($R_f$ = 0.8—0.9) leaving the desired product and unreacted L-proline starting material. This mixture is taken up in 40 ml. of acetonitrile, filtered, and then diluted with water to give a milky white mixture. This is filtered on Celite and the pad washed with water. The Celite pad is then extracted with acetonitrile and the extracts evaporated to a slurry. This is taken up in ethyl acetate, dried ($Na_2SO_4$), and evaporated to a foam. Trituration with ether gives 0.3 g. of off white solid [1(S),4S]-4-[4-[7-(aminosulfonyl)-6-chloro-3,4-dihydro-1,1-dioxo-2H-1,2,4-benzothiadiazin-3-yl]phenoxy]-1-[3-(benzoylthio)-2-methyl-1-oxopropyl]-L-proline, methyl ester as a partial ether solvate and hydrate. Tlc (silica, $R_f$ = 0.5, hexane/acetate 2:1).

| | | | | | |
|---|---|---|---|---|---|
| Anal. Calc'd. for $C_{30}H_{31}N_4O_9S_3Cl \cdot 0.5C_4H_{10} \cdot 0.75H_2O$: | C, 49.70; | H, 4.82; | N, 7.24; | S, 12.44; | Cl, 4.58 |
| Found: | C, 49.73; | H, 4,61; | N, 7.34; | S, 12.43; | Cl, 4.02. |

b) [1(S),4S]-4-[4-[7-(Aminosulfonyl)-6-chloro-3,4-dihydro-1,1-dioxo-2H-1,2,4-benzothiadiazin-3-yl]-phenoxy]-1-(3-mercapto-2-methyl-1-oxopropyl)-L-proline

The product from part (a) (300 mg., 0.4 mmole) is hydrolyzed with 40 ml. of argon purged 0.2N sodium hydroxide at 25° for 1.5 hours. The mixture is acidified with potassium bisulfate to pH 1, then extracted with ethyl acetate (2 × 75 ml.), dried ($Na_2SO_4$) and evaporated to a foam. This is triturated with ether to give a white solid; Tlc (silica; dichloromethane (8), acetic acid (1), methanol (1)) $R_f$ = 0.9, 0.6 and 0.4 with the intermediate spot being most intense.

The mixture is chromatographed on 50 g. of Sephadex (LH 20 column, 25 mm diameter), by gravity, in methanol. The $R_f$ = 0.9 material eluted first followed by the $R_f$ = 0.6 and finally the $R_f$ = 0.4 material. The $R_f$ = 0.6 compound is homogeneous by Tlc. Evaporation and trituration gives 100 mg. of crystalline solid [1(S),4S]-4-[4-[7-(aminosulfonyl)-6-chloro-3,4-dihydro-1,1-dioxo-2H-1,2,4-benzothiadiazin-3-yl]phenoxy]-1-(3-mercapto-2-methyl-1-oxopropyl]-L-proline; m.p. 197—199°.

| | | | | | | |
|---|---|---|---|---|---|---|
| Anal. Calc'd. for $C_{22}H_{25}ClN_4O_8S_3 \cdot 0.5\ H_2O$: | C, 43.02; | H, 4.27; | N, 9.12; | S, 15.66; | Cl, 5.77; | SH, 5.38 |
| Found: | C, 43.00; | H, 4.33; | N, 8.79; | S, 15.27; | Cl, 5.50; | SH, 5.16. |

## Example 6

[1(S),4S]-4-[3-[7-(Aminosulfonyl)-6-chloro-3,4-dihydro-1,1-dioxo-2H-1,2,4-benzothiadiazin-3-yl]phenoxy]-1-(3-mercapto-2-methyl-1-oxopropyl)-L-proline

a) [1(S),4S]-4-[3-[7-Aminosulfonyl)-6-chloro-3,4-dihydro-1,1-dioxo-2H-1,2,4-benzothiadiazin-3-yl]phenoxy]-1-[3-(benzoylthio)-2-methyl-1-oxopropyl]-L-proline, methyl ester

A mixture of 500 mg. (1.0 mmole) of [1(S),4S]-1-[3-(benzoylthio)-2-methyl-1-oxopropyl]-4-[3-(dimethoxydimethyl)phenoxy]-L-proline, methyl ester and 280 mg. (1.0 mmole) of 4-amino-6-chloro-1,3-benzenedisulfonamide is refluxed in 30 ml. of acetonitrile with 1 mg. of p-toluenesulfonic acid for 24 hours. The solvent is removed *in vacuo* and the residue is triturated with ethyl ether. The resulting solid is filtered off and crystallized from ethyl acetate to yield 450 mg. of [1(S),4S]-4-[3-[7-(aminosulfonyl)-6-chloro-3,4-dihydro-1,1-dioxo-2H-1,2,4-benzothiadiazin-3-yl]phenoxy]-1-[3-(benzoylthio)-2-methyl-1-oxopropyl]-L-proline, methyl ester; m.p. 135° (dec.). Tlc (silica, dichloromethane) $R_f$ = 0.7.

| | | | | | |
|---|---|---|---|---|---|
| Anal. Calc'd. for $C_{30}H_{31}ClN_4O_9S_3 \cdot 0.5\ CH_3COOC_2H_5$: | C, 50.09; | H, 4.60; | N, 7.30; | Cl, 4.62; | S, 12.53. |
| Found: | C, 50.09; | H, 4.69; | N, 7.39; | Cl, 4.67; | S, 12.36. |

b) [1(S),4S]-4-[3-[7-(Aminosulfonyl)-6-chloro-3,4-dihydro-1,1-dioxo-2H-1,2,4-benzothiadiazin-3-yl]phenoxy]-1-(3-mercapto-2-methyl-1-oxopropyl)-L-proline

To 720 mg. (1.0 mmole) of the product from part (a) under argon is added 40 ml. of 0.2 N sodium hydroxide which has been purged for 2 hours with argon. The resulting solution is stirred for 4 hours at 25°, then acidified with potassium bisulfate. Extraction with ethyl acetate, drying ($Na_2SO_4$), and evaporation gives a foam. Trituration with ether gives 400 mg. of solid 3 spots on Tlc (silica; dichloromethane (8)/acetic acid (1)/methanol (1)). Chromatography on 50 g. of Sephadex (LH20) in methanol separates the product of intermediate polarity ($R_f$ = 0.6) as the second off the column. Evaporation gives a glass which yields 170 g. of white solid [1(S),4S]-4-[3-[7-(aminosulfonyl)-6-chloro-3,4-dihydro-1,1-dioxo-2H-1,2,4-benzothiadiazin-3-yl]phenoxy]-1-(3-mercapto-2-methyl-1-oxopropyl)-L-proline; m.p. 185—188°. Trituration with ether gives an analytical sample.

| | | | | | | |
|---|---|---|---|---|---|---|
| Anal. Calc'd. for $C_{22}H_{25}ClN_4O_8S_3 \cdot 0.5\ H_2O$: | C, 43.02; | H, 4.27; | N, 9.12; | Cl, 5.77; | S, 15.66; | SH, 5.38 |
| Found: | C, 43.03; | H, 4.27; | N, 9.31; | Cl, 5.83; | S, 15.20; | SH, 5.04. |

## Example 7

[1(S),4S]-4-[3-[7-(Aminosulfonyl)-6-chloro-3,4-dihydro-1,1-dioxo-2H-1,2,4-benzothiadiazin-3-yl]phenoxy]-1-[3-(benzoylthio)-2-methyl-1-oxopropyl]-L-proline

a) [1(S),4S]-1-[3-(Benzoylthio)-2-methyl-1-oxopropyl]-4-[3-(dimethoxymethyl)phenoxy]-L-proline, diphenylmethyl ester

A mixture of 4.8 g. (9.5 mmole) of [1(S),4R]-1-[3-(benzoylthio)-2-methyl-1-oxopropyl]-4-hydroxy-L-proline, diphenylmethyl ester, 1.75 g. (1 eq.) of (3-dimethoxymethyl)phenol, and 2.73 g. (1 eq.) of triphenylphosphine in 30 ml. of dry tetrahydrofuran is stirred under argon at 0°. A solution of diethylazodicarboxylate (1.81 g./5 ml. of tetrahydrofuran) is added dropwise. After the addition is completed, the ice bath is removed and the reaction mixture is stirred at ambient temperature for 16 hours. Tlc of the reaction mixture shows a considerable amount of unreacted L-proline starting material. Therefore, an additional half equivalent of (3-dimethoxymethyl)phenol (0.88 g.), triphenylphosphine (1.37 g.) and diethylazodicarboxylate (0.9 g.) are added. After 25 hours no starting material remains.

The reaction mixture is concentrated to a residue. When the residue is slurried in petroleum ether:ether (1:1), an oil is formed. The liquid is decanted and saved. A solid forms which is filtered off and determined to be triphenylphosphine oxide. The combined solutions are concentrated to a residue.

This crude product is purified by repeated column chromatography. First, a flash chromatography on LPS silica eluting with a solvent gradient of hexane:ether (9:1 to 3:7) removes all of the dicarbethoxyhydrazine generated in the reaction and some non polar impurities. A gravity column on silica gel with an ethyl ether eluant removes the last traces of triphenylphosphine oxide. The two remaining compounds of very similar $R_f$'s are separated on a Waters Prep. LC (hexane:acetone, 4:1) giving 2.3 g. of pure [1(S),4S]-1-[3-(benzoylthio)-2-methyl-1-oxopropyl]-4-[3-(dimethoxymethyl)phenoxy]-L-proline, diphenylmethyl ester.

b) [1(S),4S]-4-[3-[7-(Aminosulfonyl)-6-chloro-3,4-dihydro-1,1-dioxo-2H-1,2,4-benzothiadiazin-3-yl]phenoxy]-1-[3-(benzoylthio)-2-methyl-1-oxopropyl]-L-proline

A solution of 1.98 g. (3.03 mmole) of the product from part (a), 0.86 g. (1 eq.) of 4-amino-6-chloro-1,3-benzenedisulfonamide, 50 mg. of p-toluenesulfonic acid, and 3 ml. of anisole in 75 ml. of acetonitrile is refluxed overnight. After the solvent is evaporated the residue is taken up in ethyl acetate and the product is extracted into 10% sodium bicarbonate. The aqueous layer is washed with ethyl acetate and acidified with 20% potassium bisulfate. The product is extracted into ethyl acetate. The organic phase is washed with water and brine, dried ($Na_2SO_4$), and evaporated to a solid. Trituration with dichloromethane removes most of a less polar impurity. Final purification on a Sephadex LH20 column eluting with methanol followed by evaporation of the solvent and trituration with ethyl ether gives [1(S),4S]-4-[3-[7-(aminosulfonyl)-6-chloro-3,4-dihydro-1,1-dioxo-2H-1,2,4-benzothiadiazin-3-yl]phenoxy]-1-[3-(benzoylthio)-2-methyl-1-oxopropyl]-L-proline as a white crystalline solid which is homogeneous by Tlc.

| | | | | | |
|---|---|---|---|---|---|
| Anal. Calc'd. for $C_{29}H_{29}ClN_4O_9S_3 \cdot 1\ H_2O$: | C, 47.89; | H, 4.30; | N, 7.70; | S, 13.23; | Cl, 4.87 |
| Found: | C, 47.98; | H, 4.33; | N, 7.70; | S, 13.10; | Cl, 4.90. |

## Example 8

[1(S),4S]-4-[4-[7-(Aminosulfonyl)-6-chloro-3,4-dihydro-1,1-dioxo-2H-1,2,4-benzothiadiazin-3-yl]phenoxy]-1-[3-(benzoylthio)-2-methyl-1-oxopropyl]-L-proline

A slurry of 1.0 g. (1.5 mmole) of [1(S),4S]-1-[3-(benzoylthio)-2-methyl-1-oxopropyl]-4-[4-(dimethoxymethyl)phenoxy]-L-proline, diphenylmethyl ester, 0.5 g. (1.75 mmole) of 4-amino-6-chloro-1,3-benzenedisulfonamide, 50 mg. of p-toluenesulfonic acid, and 1.5 ml. of anisole in 25 ml. of dry acetonitrile is refluxed for 5 hours, then evaporated to a foam. This is partitioned between ethyl acetate and dilute sodium bicarbonate. The aqueous phase is acidified and extracted with ethyl acetate. Drying ($Na_2SO_4$) and evaporation gives 0.6 g. of a tan foam, consisting of 3 components by Tlc [$R_f$ = 0.35, 0.5, 0.6 on silica; ethyl acetate (120), pyridine (20), acetic acid (6), water (1)]. Chromatography on 200 g. of Sephadex (LH20) in methanol affords on evaporation of the $R_f$ = 0.35 fractions and trituration with ether, 200 mg. of crystalline [1(S),4S]-4-[4-[7-(aminosulfonyl)-6-chloro-3,4-dihydro-1,1-dioxo-2H-1,2,4-benzothiadiazin-3-yl]phenoxy-1-[3-(benzoylthio)-2-methyl-1-oxopropyl]-L-proline; m.p. 175—185°.

| | | | | | |
|---|---|---|---|---|---|
| Anal. Calc'd. for $C_{29}H_{29}N_4O_9S_3Cl \cdot 0.5\ H_2O$: | C, 48.50; | H, 4.21; | N, 7.80; | S, 13.39; | Cl, 4.94 |
| Found: | C, 48.39; | H, 4.35; | N, 7.62; | S, 13.32; | Cl, 4.95. |

## Example 9

[1(S),4S]-4-[3-[6-(Aminosulfonyl)-7-chloro-1,2,3,4-tetrahydro-4-oxo-2-quinazolinyl]phenoxy]-1-[3-(benzoylthio)-2-methyl-1-oxopropyl]-L-proline

A mixture of 1.7 g. (2.6 mmole) of [1(S),4S]-1-[3-(benzoylthio)-2-methyl-1-oxopropyl]-4-[3-(dimethoxymethyl)phenoxy]-L-proline, diphenylmethyl ester, 0.75 g. (3.0 mmole) of 2-amino-1-chloro-5-sulfamoylbenzamide, 50 mg. of p-toluene-sulfonic acid, and 3 ml. of anisole in 100 ml. of dry acetonitrile is refluxed for 2 hours, then evaporated and the residue shaken with ethyl acetate and aqueous sodium bicarbonate. A gummy solid formed, and 500 ml. of water is necessary for its solubilization. The organic layer is discarded and the aqueous layer is acidified with potassium bisulfate and extracted with ethyl acetate. The extracts are dried ($Na_2SO_4$) and evaporated to a solid with one major ($R_f$ = 0.32) and one minor ($R_f$ = 0.50) component by Tlc [silica; ethyl acetate (120), pyridine (20), acetic acid (6), water (11)]. Purification on a 200 g. Sephadex (LH 20) column in methanol gives, on evaporation and trituration with ether, 0.9 g. of white solid [1(S),4S]-4-[3-[6-(aminosulfonyl)-7-chloro-1,2,3,4-tetrahydro-4-oxo-2-quinazolinyl]phenoxy]-1-[3-(benzoylthio)-2-methyl-1-oxopropyl]-L-proline; m.p. 173—178°, essentially homogeneous by Tlc.

| | | | | | |
|---|---|---|---|---|---|
| Anal. Calc'd. for $C_{30}H_{29}ClN_4O_8S_2 \cdot 0.5\ H_2O$: | C, 52.82; | H, 4.43; | N, 8.21; | S, 9.40; | Cl, 5.20 |
| Found: | C, 53.00; | H, 4.54; | N, 8.19; | S, 9.46; | Cl, 5.41. |

Example 10

[1(S),4S]-4-[2-[6-(Aminosulfonyl)-7-chloro-1,2,3,4-tetrahydro-4-oxo-2-quinazolinyl]phenoxy]-1-[3-(benzoylthio)-2-methyl-1-oxopropyl]-L-proline

a) [1(S),4S]-1-[3-(Benzoylthio)-2-methyl-1-oxopropyl]-4-[2-(dimethoxymethyl)phenoxy]-L-proline, diphenylmethyl ester

To a stirred mixture of 5.04 g. (0.01 mole) of [1(S),4R]-1-[3-(benzoylthio)-2-methyl-1-oxopropyl]-4-hydroxy-L-proline, diphenylmethyl ester, 2.52 g. (1.5 eq.) of 2-(dimethoxymethyl)phenol, and 3.93 g. (1.5 eq.) of triphenylphosphine in 15 ml. of dry tetrahydrofuran under argon at 0° is added dropwise a solution of 2.61 g. (1.5 eq.) of diethylazodicarboxylate in 5 ml. of tetrahydrofuran. The reaction mixture is stirred at ambient temperature for approximatley 20 hours. The reaction mixture is concentrated to a residue and chromatographed twice [silica gel gravity column (ethyl ether eluent) and LPS-1 silica (hexane:acetone, 4:1) yielding [1(S),4S]-1-[3-(benzoylthio)-2-methyl-1-oxopropyl]-4-[2-(dimethoxymethyl)phenoxy]-L-proline, diphenylmethyl ester

b) [1(S),4S]-4-[2-[6-(Aminosulfonyl-7-chloro-1,2,3,4-tetrahydro-4-oxo-2-quinazolinyl]phenoxy]-1-[3-(benzoylthio)-2-methyl-1-oxopropyl}-L-proline

1 g. (1.5 mmole) of the product from part (a), 0.5 g. (2 mmole) of 2-amino-4-chloro-5-sulfamylbenzamide and 2 ml of anisole are combined in 15 ml. of acetonitrile. A catalytic amount of p-toluenesulfonic acid (50 mg.) is added and the reaction mixture is refluxed for 2 hours. After concentrating to a residue, the reaction mixture is dissolved in ethyl acetate and water. The aqeous layer is discarded and the product is extracted into a sodium bicarbonate solution. After washing with ethyl acetate, the aqueous phase is acidified with potassium bisulfate and the product is extracted with ethyl acetate. The ethyl acetate solution was washed with water and brine and dried ($MgSO_4$). The solvent is evaporated to give a white solid which is purified on a Sephadex (LH20) column. Trituration of the chromatographed product gives [1(S),4S]-4-[2-[6-(aminosulfonyl)-7-chloro-1,2,3,4-tetrahydro-4-oxo-2-quinazolinyl]phenoxy]-1-[3-(benzoylthio)-2-methyl-1-oxopropyl]-L-proline; m.p. 190—205°, as a white crystalline solid.

| | | | | | |
|---|---|---|---|---|---|
| Anal. Calc'd. for $C_{30}H_{29}ClN_4O_8S_2 \cdot 0.38\ H_2O$: | C, 52.99; | H, 4.41; | N, 8.24; | S, 9.43; | Cl, 5.21 |
| Found: | C, 52.99; | H, 4.43; | N, 8.12; | S, 9.08; | Cl, 5.28. |

Example 11

(cis)-4-[3-[6-(Aminosulfonyl)-7-chloro-1,2,3,4-tetrahydro-4-oxo-2-quinazolinyl]phenoxy]-1-[[(3-ethoxy-3-oxopropyl)ethylamino]carbonyl]-L-proline

a) (trans)-1-[(1,1-Dimethylethoxy)carbonyl]-4-hydroxy-L-proline

To a solution of 20 g. (153 mmole) of (trans)-4-hydroxy-L-proline in 150 ml. of aqueous acetone is added triethylamine (32 ml., 1.5 eq.) followed by 2-(tert-butoxycarbonyloxyimino)-2-phenylacetonitrile (41.3 g., 1.1 eq.) and the resulting solution is stirred at room temperature. After 18 hours, the solution is diluted with water and extracted twice with ether. The ether fractions are discarded. The aqueous layer is acidified with 300 ml. of 0.5 M citric acid and extracted four times with ethyl acetate. The original fractions are combined and washed with water and brine. After drying ($Na_2SO_4$), the solvent is removed at reduced pressure to give 26.55 g. of (trans)-1-[(1,1-dimethylethoxy)carbonyl]-4-hydroxy-L-proline as a pale yellow oil, $R_f$ (silica gel, dichloromethane/methanol/acetic acid; 18:1:1) = 0.64.

b) (trans)-1-[(1,1-Dimethylethoxy)carbonyl]-4-(1,3-dioxobutoxy)-L-proline

To a suspension of 13 g. (56 mmole) of (trans)-1-[(1,1-dimethylethoxy)carbonyl]-4-hydroxy-L-proline in 100 ml. of anhydrous dichloromethane under argon is added triethylamine (7.81 ml., 1 eq.). To the resulting clear solution is added distilled diketene (4.39 ml., 1 eq.) and the solution darkened. After stirring at room temperature for 3.5 hours, the solvent is removed at reduced pressure. The residue is dissolved in ethyl acetate and washed with 1M hydrochloric acid (60 ml.) and water. After drying ($Na_2SO_4$), the solvent is removed at reduced pressure to give 13.92 g. of (trans)-1-[(1,1-dimethylethoxy)carbonyl]-4-(1,3-dioxobutoxy)-L-proline as a yellow oil. $R_f$ (silica gel, dichloromethane/methanol/acetic acid; 8:1:1) = 0.58, secondary spot at $R_f$ = 0.7.

c) (trans)-1-[1,1-Dimethylethoxy)carbonyl]-4-(1,3-dioxobutoxy)-L-proline, phenylmethyl ester

To a solution of 13.92 g. (44 mmole) of (trans)-1-[(1,1-dimethylethoxy)carbonyl]-4-(1,3-dioxobutoxy)-L-proline in 100 ml. of dimethylformamide (distilled in glass) under argon is added sodium bicarbonate (7.39 g., 2 eq.) followed by benzyl bromide (26.17 ml., 5 eq.). The resulting solution is stirred for 24 hours at room temperature. The solution is diluted with water and extracted three times with ether. The ether extracts are combined and washed with water, 1N sodium bicarbonate (twice), water, and brine. After drying ($MgSO_4$), the solvent is removed at reduced pressure to give 38.79 g. of crude (trans)-1-[(1,1-dimethylethoxy)carbonyl]-4-(1,3-dioxobutoxy)-L-proline, phenylmethyl ester as a yellow oil. $R_f$ (silica gel, ethyl acetate) = 0.61, secondary spots at $R_f$ = 0.13, 0.45, 0.65.

d) (trans)-1-[(1,1-Dimethylethoxy)carbonyl]-4-hydroxy-L-proline, phenylmethyl ester

To a solution of the crude (trans)-1-[(1,1-dimethylethoxy)carbonyl]-4-(1,3-dioxobutoxy)-L-proline, phenylmethyl ester (34.79 g.) in 500 ml. of isopropanol is added a solution of hydrazine hydrate (3.83 ml., 2

eq.) in 50 ml. of isopropanol. The resulting solution is stirred under argon for four hours. The solvent is removed at reduced pressure and the residue taken up in ethyl acetate. This is washed with water (twice), ice cold 1N hydrochloric acid, 1N sodium bicarbonate and brine. After drying ($MgSO_4$), the solvent is removed at reduced pressure. The residue is flash chromatographed (Whatman silica gel LPS-1, ethyl acetate:hexane, 6:4) to give 9.53 g. of (trans)-1-[(1,1-dimethylethoxy)carbonyl]-4-hydroxy-L-proline, phenylmethyl ester as a pale yellow oil, $R_f$ (silica gel, ethyl acetate) = 0.45.

e) (trans)-4-Hydroxy-L-proline, phenylmethyl ester, p-toluenesulfonic acid salt

75 ml. of cold (0°) trifluoroacetic acid is added to (trans)-1-[(1,1-dimethylethoxy)carbonyl]-4-hydroxy-L-proline, phenylmethyl ester (9.5 g., 29.56 mmole) and the resulting solution is stirred at 0° for one hour. The solvent is removed at reduced pressure. Toluene is added and the solution is concentrated once more. The residue is dissolved in ether and to it is added a solution of p-toluenesulfonic acid (5.62 g., 1 eq.) in ether. A precipitate slowly forms. This is filtered and washed with ether to give 9.33 g. of (trans)-4-hydroxy-L-proline, phenylmethyl ester, p-toluenesulfonic acid salt as a white solid; m.p. 120—124°.

f) 3-(Ethylamino)propanoic acid, ethyl ester

Into 200 ml. of cold (−60°) absolute ethanol is passed anhydrous ethylamine (36 g., 781 mmole), 1.1 eq.). To the resulting ethanolic solution is added dropwise a solution of ethyl acrylate (71 g., 710 mmole, 1 eq.) in 150 ml. of absolute ethanol over a period of several hours. The resulting mixture is then stirred at −60° for 0.5 hours and allowed to warm slowly to room temperature where it is stirred for another 20 hours. The solvent is removed at reduced pressure to give 33 g. of 3-(ethylamino)propanoic acid, ethyl ester as colorless liquid; b.p. 80—83°.

g) (trans)-1-[[(3-Ethoxy-3-oxopropyl)ethylamino]carbonyl]-4-hydroxy-L-proline, phenylmethyl ester

To a cold (−30°) solution of 1.25 M phosgene in benzene (25.52 ml., 1.5 eq.) in 20 ml. of anhydrous dichloromethane under argon is added dropwise a solution of 3-(ethylamino)propanoic acid, ethyl ester (3.09 g., 21.26 mmole) and N-methylmorpholine (3.5 ml., 1.5 eq.) in 20 ml. of dichloromethane. The resulting mixture is stirred for one hour at −30° and then for one hour at room temperature. The solvent is removed at reduced pressure. Additional dichloromethane is added and the solution is concentrated once more.

This residue is dissolved in 30 ml. of dichloromethane and (trans)-4-hydroxy-L-proline, phenylmethyl ester, p-toluenesulfonic acid salt (10.5 g., 1.2 eq.) is added. To the resulting suspension is added dropwise N-methylmorpholine (5.6 ml., 2.4 eq.) in 20 ml. of dichloromethane. The resulting mixture is stirred at room temperature for 15 hours. The solvent is removed at reduced pressure and the residue taken up in ethyl acetate. This is washed with water (twice), 1N hydrochloric acid (twice), 1N sodium bicarbonate (twice), and once with brine. After drying ($MgSO_4$), the solvent is removed at reduced pressure. The residue is flash chromatographed (Whatman silica gel LPS-1, hexane:ethyl acetate; 65:35) to give 7.72 g. of (trans)-1-[[(3-ethoxy-3-oxopropyl)ethylamino]carbonyl]-4-hydroxy-L-proline, phenylmethyl ester as a colorless oil, $R_f$ (silica gel, ethyl acetate) = 0.34.

h) (cis)-4-[3-(Dimethoxymethyl)phenoxy]-1-[[(3-ethoxy-3-oxopropyl)ethylamino]carbonyl]-L-proline, phenylmethyl ester

To a cold (0°) solution of 3-(dimethoxymethyl)phenol (4.96 g., 1.5 eq.), (trans)-1-[[(3-ethoxy-3-oxopropyl)ethylamino]carbonyl]-4-hydroxy-L-proline, phenylmethyl ester (7.72 g., 19.67 mmole), and triphenylphosphine (7.74 g., 1.5 eq.) in 100 ml. of anhydrous tetrahydrofuran under argon is added dropwise a solution of diethylazodicarboxylate in 20 ml. of tetrahydrofuran. Following the addition, the solution is stirred for one hour at 0° and for 18 hours at room temperature thereafter. The solution is diluted with ether and filtered. The filtrate is washed twice with water and dried ($MgSO_4$). The solvent is removed at reduced pressure. The residue is flash chromatographed (Whatman silica gel LPS-1; hexane:acetone; 7:3) and the resulting product is taken up in about 100 ml. of a 1:1 solution of petroleum ether and ether and allowed to stand overnight under refrigeration. The solution is filtered through glass wool and the solvent is removed at reduced pressure. The residue is taken up in benzene and run through the Waters Prep liquid chromatography system (hexane:acetone, 4:1) to give 3.11 g. of (cis)-4-[3-(dimethoxymethyl)-phenoxy]-1-[[(3-ethoxy-3-oxopropyl)ethylamino]carbonyl]-L-proline, phenylmethyl ester as a clear oil, $R_f$ (silica gel, hexane:acetone; 1:1) = 0.36.

i) (cis)-4-[3-[6-(Aminosulfonyl)-7-chloro-1,2,3,4-tetrahydro-4-oxo-2-quinazolinyl]phenoxy]-1-[[(3-ethoxy-3-oxopropyl)ethylamino]carbonyl]-L-proline, phenylmethyl ester

A mixture of (cis)-4-[3-(dimethoxymethyl)phenoxy]-1-[[(3-ethoxy-3-oxopropyl)ethylamino]carbonyl]-L-proline, phenylmethyl ester (700 mg., 1.29 mmole), 2-amino-4-chloro-5-sulfamylbenzamide (290 mg., 1.16 mmole) and approximately 10 mg. of aqueous p-toluenesulfonic acid in 12 ml. of dry acetonitrile is heated at reflux. After 75 minutes the mixture is cooled, solvent removed at reduced pressure and the residue treated with ether to yield a crystalline solid. This material is flash chromatographed on silica gel eluted with hexane:acetone (4:6) and approximately 50 ml. fractions are collected. Fractions #27—40 are combined and concentrated to give 790 mg. of (cis)-4-[3-[6-(aminosulfonyl)-7-chloro-1,2,3,4-tetrahydro-4-

oxo-2-quinazolinyl]phenoxy]-1-[[(3-ethoxy-3-oxopropyl)ethylamino]carbonyl]-L-proline, phenylmethyl ester.

j) (cis)-4-[3-[6-(Aminosulfonyl)-7-chloro-1,2,3,4-tetrahydro-4-oxo-2-quinazolinyl]phenoxy]-1-[[(3-ethoxy-3-oxopropyl)ethylamino]carbonyl]-L-proline

To a solution of the phenylmethyl ester product of part (i) (505 mg., 0.69 mmole) in 40 ml. of absolute ethanol is added approximately 70 mg. of 10% palladium on charcoal. The resulting mixture is stirred under hydrogen for approximately 2.5 hours at which time Tlc analysis shows no starting material remaining. The mixture is filtered and concentrated at reduced pressure to give a pale gray foam which is dried under vacuum overnight to give 430 mg. of material. In order to remove additional palladium/charcoal, this material is dissolved in ethanol, millipore filtered, and concentrated at reduced pressure. The residue is dissolved in tetrahydrofuran, precipitated with ether to give a white solid, and dried overnight under vacuum to give (cis)-4-[3-[6-(aminosulfonyl)-7-chloro-1,2,3,4-tetrahydro-4-oxo-2-quinazolinyl]phenoxy]-1-[[(3-ethoxy-3-oxopropyl)ethylamino]carbonyl]-L-proline.

| | | | | | |
|---|---|---|---|---|---|
| Anal. Calc'd. for $C_{27}H_{32}N_5O_9SCl\cdot0.5H_2O$: | C, 50.11; | H, 514; | N, 10.82; | S, 4.95; | Cl, 5.48 |
| Found: | C, 50.06; | H, 5.22; | N, 10.51; | S, 4.98; | Cl, 5.41. |

## Example 12

(cis)-4-[3-[6-(Aminosulfonyl)-7-chloro-1,2,3,4-tetrahydro-4-oxo-2-quinazolinyl]phenoxy]-1-[[(3-hydroxy-3-oxopropyl)ethylamino]carbonyl]-L-proline

The product from Example 11 is dissolved in aqueous tetrahydrofuran and treated with a molar excess of aqueous sodium hydroxide. After the reaction is complete, the solution is acidified with dilute hydrochloric acid to precipitate out (cis)-4-[3-[6-(aminosulfonyl)-7-chloro-1,2,3,4-tetrahydro-4-oxo-2-quinazolinyl]phenoxy]-1-[[(3-hydroxy-3-oxopropyl)ethylamino]carbonyl]-L-proline.

## Example 13

[1(S),4S]-4-[4-[7-(Aminosulfonyl)-3,4-dihydro-1,1-dioxo-6-(trifluoromethyl-2H-1,2,4-benzothiadiazin-3-yl]phenoxy]-1-[3-(benzoylthio)-2-methyl-1-oxopropyl]-L-proline

To a mixture of [1(S),4S]-1-[3-(benzoylthio)-2-methyl-1-oxopropyl]-4-[4-(dimethoxymethyl)phenoxy]-L-proline, diphenylmethyl ester (3.5 g., 5.35 mmole), 4-amino-6-trifluoromethyl-1,3-benzenedisulfonamide (1.75 g.) and anisole (3.5 ml.) in 80 ml. acetonitrile is added p-toluenesulfonic acid (120 mg.). The reaction mixture is refluxed for 17 hours. After concentrating to a residue and triturating with dichloromethane, the crude solid is flash chromatographed on LPS-1 silica eluting with ethyl acetate:pyridine:acetic acid:water (2400:50:15:27). The fractions containing the pure product are combined and concentrated. Further purification is achieved on an LH-20 column eluting with methanol to give white solid [1(S),4S]-4-[4-[7-(aminosulfonyl)-3,4-dihydro-1,1-dioxo-6-(trifluoromethyl-2H-1,2,4-benzothiadiazin-3-yl]phenoxy]-1-[3-(benzoylthio)-2-methyl-1-oxopropyl]-L-proline; m.p. 160° (foams); $[\alpha]_D$ = −27° (c = 1, methanol). $R_f$ (silica gel, dichloromethane/methanol/acetic acid; 8:1:1) = 0.42.

| | | | | | |
|---|---|---|---|---|---|
| Anal. Calc'd. for $C_{30}H_{29}F_3N_4O_9S_3\cdot1\ H_2O\cdot.0.7\ C_2H_5OC_2H_5$: | C, 48.77; | H, 4.53; | N, 6.89; | S, 11.83; | F, 7.01 |
| Found: | C, 48.36; | H, 4.27; | N, 7.15; | S, 11.79; | F, 7.50. |

## Example 14

[1(S),4S]-4-[3-[7-(Aminosulfonyl)-3,4-dihydro-1,1-dioxo-6-(trifluoromethyl)-2H-1,2,4-benzothiadiazin-3-yl]phenoxy]-1-[3-(benzoylthio)-2-methyl-1-oxopropyl]-L-proline

To a mixture of [1(S),4S]-1-[3-(benzoylthio)-2-methyl-1-oxopropyl]-4-[3-(dimethoxymethyl)phenoxy]-L-proline, diphenylmethyl ester (6.0 g., 9 mmole), 4-amino-6-trifluoromethyl-1,3-benzenedisulfonamide (3 g., 10 mmole) and anisole (3 ml.) in 150 ml. of acetonitrile is added p-toluenesulfonic acid (100 mg.). The mixture is refluxed for 12 hours. After evaporating to an oil, the crude product is taken up in ethyl acetate, washed with water, dried ($Na_2SO_4$), and evaporated to a solid. The product is flash chromatographed on LPS-1 silica eluting with ethyl acetate:pyridine:acetic acid:water (2400:50:13:27). The fractions containing the pure product are combined and concentrated. Further purification is achieved on an LH-20 column eluting with methanol to give white solid [1(S),4S]-4-[3-[7-(aminosulfonyl)-3,4-dihydro-1,1-dioxo-6-(trifluoromethyl)-2H-1,2,4-benzothiadiazin-3-yl]phenoxy]-1-[3-(benzoylthio)-2-methyl-1-oxopropyl]-L-proline; m.p. 175—178° (foams); $[\alpha]_D$ = −30.4° (c = 1.0, methanol).

$R_f$ (silica gel; dichloromethane:methanol:acetic acid; 8::1:1) = 0.45.

| | | | | | |
|---|---|---|---|---|---|
| Anal. Calc'd. for $C_{30}H_{29}F_3N_4O_9S_3$: | C, 48.51; | H, 3.94; | N, 7.54; | S, 12.95; | F, 7.67 |
| Found: | C, 48.54; | H, 4.13; | N, 7.23; | S, 12.69; | F, 7.50. |

## Example 15

[1(S),4S]-4-[4-[7-(Aminosulfonyl)-6-chloro-3,4-dihydro-2-methyl-1,1-dioxo-2H-1,2,4-benzothiadiazin-3-yl]phenoxy]-1-[3-(benzoylthio)-2-methyl-1-oxopropyl]-L-proline

a) [1(S),4S]-1-[3-(Benzoylthio)-2-methyl-1-oxopropyl]-4-(4-formylphenoxy)-L-proline

A mixture of [1(S),4R]-1-[3-(benzoylthio)-2-methyl-1-oxopropyl]-4-hydroxy-L-proline, diphenylmethyl ester (10 g., 20 mmole), 4-hydroxybenzaldehyde (2.4 g., 20 mmole), and triphenylphosphine (5.2 g., 20

mmole) in benzene (150 ml.) is evaporated to dryness, then taken up in dry, freshly distilled tetrahydrofuran (100 ml.), cooled to −8° and treated with diethylazodicarboxylate (3.6 g., 20 mmole). After 15 minutes the cooling bath is replaced with a cold water bath and the temperature is raised to 50—52° over 2 hours and is held there for another 2 hours, before allowing it to come to 25° overnight. The oil obtained on evaporation is taken in dichloromethane (100 ml.), treated with trifluoroacetic acid (50 ml.), and allowed to stand at 25° for 3 hours. The volatiles are evaporated and toluene is added and evaporated. This treatment is repeated twice to give an oil with no acidic odor. This material is taken up in ethyl acetate and extracted with saturated sodium bicarbonate (2 × 75 ml.). The aqueous fraction is washed with ethyl acetate, acidified with concentrated hydrochloric acid, extracted with ethyl acetate (500 ml.), dried ($Na_2SO_4$), and evaporated to give 11 g. of viscous oil containing product and deprotected starting material. Most of the latter impurity is removed by slurrying in dichloromethane (300 ml.) and filtering. The filtrates are applied to an 800 g. silica (60—200 mesh) dry column and eluted with acetone. The eluant is somewhat colored. It is evaporated and the resultant oil is taken up in ethyl acetate (200 ml.) and treated with excess dicyclohexylamine. After standing undisturbed for 3 hours, the yellow solution is decanted and the residual solid is washed with more solvent, filtered, and dried to give 6 g. of beige [1(S),4S]-1-[3-(benzoylthio)-2-methyl-1-oxopropyl]-4-(4-formylphenoxy)-L-proline, dicyclohexylamine salt.

This salt product is shaken with 10% potassium bisulfate and ethyl acetate, the organic layer is dried and evaporated to a tan foam. This material is covered with a small volume of ethyl acetate and scratched. The resulting white solid is filtered, washed with a small volume of cold ethyl acetate, then with hexane, dried at 55° *in vacuo* for 5 hours to give 1.8 g. of white solid [1(S),4S]-1-[3-(benzoylthio)-2-methyl-1-oxopropyl]-4-(4-formylphenoxy]-L-proline; m.p. 138—140°; $[\alpha]_D$ = −35.8° (c = 1.0, methanol). $R_f$ (silica gel; dichloromethane:methanol; 4:1) = 0.6.

| | | | | |
|---|---|---|---|---|
| Anal. Calc'd. for $C_{23}H_{23}NO_6S$: | C, 62.57; | H, 5.25; | N, 3.17; | S, 7.26 |
| Found: | C, 62.30; | H, 5.34; | N, 3.13; | S, 7.03. |

b) 4-Amino-6-chloro-$N^3$-methyl-1,3-benzene-disulfonamide

A mixture of 4-amino-6-chloro-1,3-benzene-disulfonammide (30 g., 105 mmole) and urea (12.6 g., 210 mmole) are ground together and then heated in a 200—205° oil bath. Within 30 minutes the sample is a molten foam, which in another 30 minutes resolidifies. After a further 30 minutes heating, the flask is cooled and the contents slurried in water and acidified. The cream-colored powder is filtered, washed with water, and dried azeotropically with benzene to yield 31 g. of 6-chloro-3,4-dihydro-3-oxo-2H-1,2,4-benzothiadiazine-7-sulfonamide, 1,1-dioxide.

Under argon, this 6-chloro-3,4-dihydro-3-oxo-2H-1,2,4-benzothiadiazine-7-sulfonamide, 1,1-dioxide (10 g., 30 mmole) is dissolved in dimethylformamide (25 ml.) at 60—65°. To this is added 1 equivalent of sodium hydride (60% in mineral oil, 1.24 g., 30 mmole) in portions. After heating and stirring for 15 minutes, methyl iodide (4.2 g., 30 mmole) is dripped in over 5 minutes. The mixture is then heated at 60—65° for one hour. The mixture is cooled somewhat and added to cold water (800 ml.), giving beige crystals and a yellow aqueous material. The solid is washed with water and air-dried to give 9.2 g. of 6-chloro-3,4-dihydro-2-methyl-3-oxo-2H-1,2,4-benzothiadiazepin-7-sulfonamide, 1,1-dioxide.

The above 6-chloro-3,4-dihydro-2-methyl-3-oxo-2H-1,2,4-benzothiadiazepine-7-sulfonamide, 1,1-dioxide (9.2 g., 28 mmole) is dissolved in 20% sodium hydroxide (90 ml.) and refluxed overnight in a 120—140° oil bath. The clear solution goes from brown to yellow. The mixture is diluted with water (350 ml.) and extracted with hexane. The aqueous phase is acidified with concentrated hydrochloric acid and chilled at 5° for 36 hours. The resulting beige solid is filtered, washed with water, and dried azeotropically with benzene to give 8.0 g. of crude product. Recrystallization of 6 g. of this material from ethanol (100 ml.) and water (200 ml.) gives 4 g. of 4-amino-6-chloro-$N^3$-methyl-1,3-benzene-disulfonamide as off-white crystals; m.p. 168—170°. [Literature m.p. 168-169°, *JACS*, Vol. 82, p. 1132 (1960)].

c) [1(S),4S]-4-[4-[7-(Aminosulfonyl)-6-chloro-3,4-dihydro-2-methyl-1,1-dioxo-2H-1,2,4-benzothiadiazin-3-yl]phenoxy]-1-[3-(benzoylthio)-2-methyl-1-oxopropyl]-L-proline

A mixture of [1-(S),4S]-1-[3-(benzoylthio)-2-methyl-1-oxopropyl]-4-(4-formylphenoxy)-L-proline (1.5 g., 3.4 mmole) and 4-amino-6-chloro-$N^3$-methyl-1,3-benzenedisulfonamide (1.02 g., 3.4 mmole) in acetonitrile (200 ml.) is treated with 50 mg. of p-toluenesulfonic acid and distilled to a final volume of 50 ml., in portions, over 36 hours. Evaporation gives a foam which is insoluble in ethyl acetate. Chromatography on 200 g. of LH-20 Sephadex in methanol removes the color but gives incomplete separation of product from reactants. The product containing fractions are pooled, evaporated and chromatographed on 800 g. silica (60—200 mesh) column in ethyl acetate (360), pyridine (20), water (11), and acetic acid (6). Pooling of the product containing fractions gives a gum. This is taken up in methanol and chromatographed on 200 g. LH-20. Product containing fractions are pooled and evaporated to a glass. Trituration with ether gives 0.3 g. of off-white solid [1(S),4S]-4-[4-[7-(aminosulfonyl)-6-chloro-3,4-dihydro-2-methyl-1,1-dioxo-2H-1,2,4-benzothiadiazin-3-yl]phenoxy]-1-[3-(benzoylthio)-2-methyl-1-oxopropyl]-L-proline; m.p. 178—184°; $[\alpha]_D$ = −25° (c = 0.1, methanol). Drying *in vacuo* gives an analytical sample. $R_f$ (silica; dichloromethane: methanol:acetic acid; 8:1:1) = 0.43.

| | | | | | |
|---|---|---|---|---|---|
| Anal. Calc'd. for $C_{30}H_{31}ClN_4O_9S_3 \cdot 1.0\ H_2O$: | C, 48.61; | H, 4.49; | N, 7.56; | S, 12.98; | Cl, 4.78 |
| Found: | C, 49.00; | H, 4.45; | N, 7.35; | S, 13.05; | Cl, 4.71. |

Examples 16—28

Following the procedures of Examples 1 to 10 and 13 to 15 the dimethoxymethyl or formyl substituted phenoxy proline ester of Col. I is reacted with the substituted benzeneamine shown in Col. II to yield the ester product of Col. III. If R is methyl, treatment with sodium hydroxide as described in Examples 1 to 3, 5 and 6 yields the mercaptoalkanoyl-L-proline shown in Col. IV. If R is benzhydryl, treatment with p-toluenesulfonic acid and anisole yields the acylmercaptoalkanoyl-L-proline shown in Col. V.

Col. I

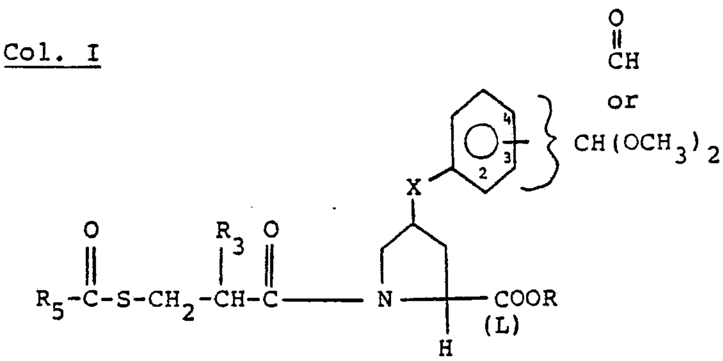

Col. II

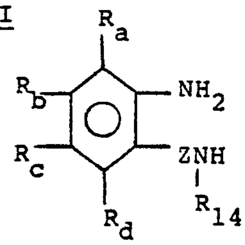

Col. III

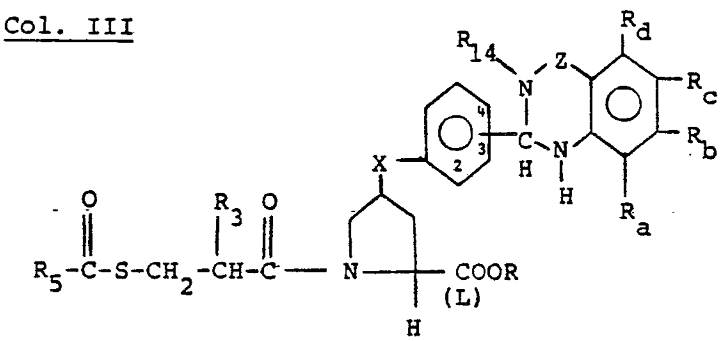

Col. IV

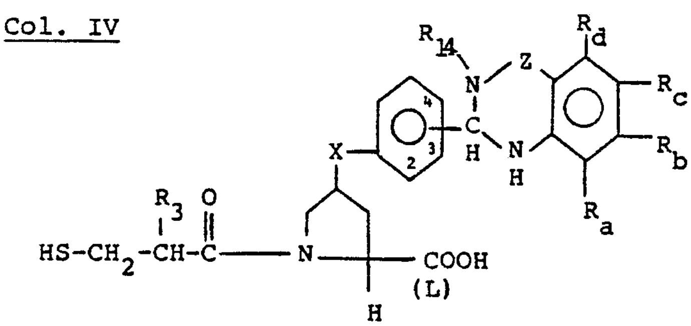

Col. V

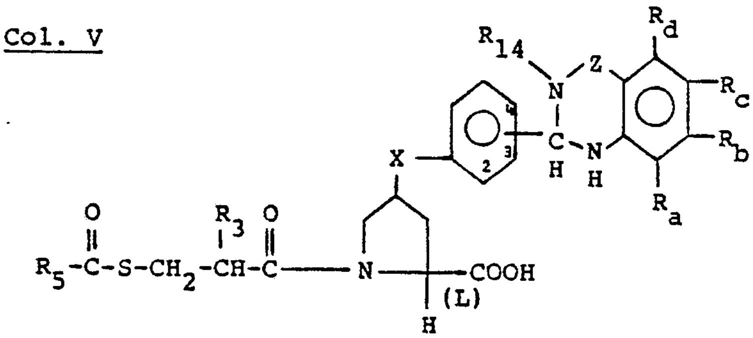

| Example | X | $R_5$ | $R_3$ | R | Position | $R_a$ | $R_b$ | $R_c$ | $R_d$ | $R_{14}$ | Z |
|---|---|---|---|---|---|---|---|---|---|---|---|
| 16 | O | $H_3C-$ | $H_3C-$ | $-CH_3$ | 2– | H | Cl | $SO_2NH_2$ | H | H | CO |
| 17 | S | $H_3C-$ | $H_3C-$ | 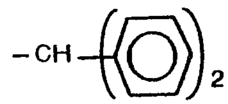 | 3– | H | Cl | $SO_2NH_2$ | H | H | CO |
| 18 | O | $H_3C-$ | $H_3C-$ | 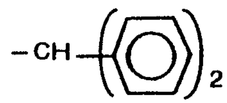 | 4– | H | Cl | $SO_2NH_2$ | H | 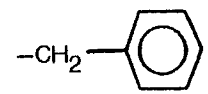 | $SO_2$ |
| 19 | S | 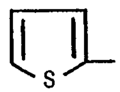 | H– | 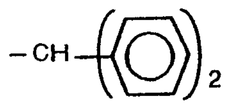 | 2– | H | H | H | H | H | $SO_2$ |
| 20 | O | 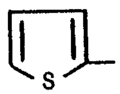 | $H_5C_2-$ | $-CH_3$ | 3– | $H_3C$ | H | H | H | H | CO |
| 21 | O | 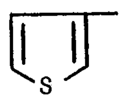 | $H_3C-$ | 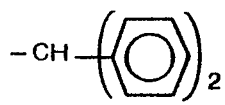 | 4– | H | $OC_2H_5$ | $SO_2NH_2$ | H | H | $SO_2$ |
| 22 | O | 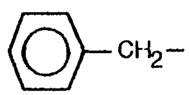 | $F_3C$ | $-CH_3$ | 2– | H | Cl | $SO_2NH_2$ | H | $-CH_3$ | $SO_2$ |
| 23 | O | 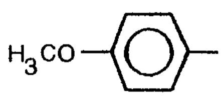 | $H_3C-$ | $-CH_3$ | 3– | H | $CF_3$ | $NO_2$ | H | H | CO |
| 24 | O | 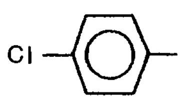 | $H_3C-$ | 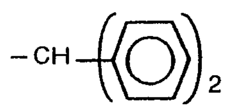 | 4– | H | Cl | $NO_2$ | H | H | CO |

| Example | X | $R_5$ | $R_3$ | R | Position | $R_a$ | $R_b$ | $R_c$ | $R_d$ | $R_{14}$ | Z |
|---|---|---|---|---|---|---|---|---|---|---|---|
| 25 | S | $H_5C_2-$ | $H_3C-$ | $-CH_3$ | 2– | H | $SO_2NH_2$ | Cl | H | $-CH_3$ | $SO_2$ |
| 26 | O | 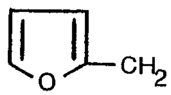 | $H_3C-$ | $-CH_3$ | 3– | Cl | H | H | $SO_2NH_2$ | H | CO |
| 27 | O | 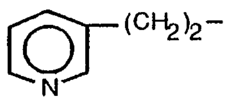 | $H_3C-$ | 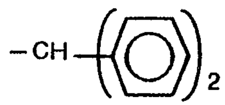 | 4– | H | Br | $SO_2NH_2$ | H | H | CO |
| 28 | O | 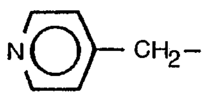 | $H_3C-$ | 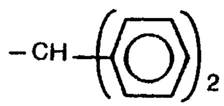 | 2– | H | $NO_2$ | $SO_2NH_2$ | H | H | $SO_2$ |

Examples 29—40

Following the procedure of Example 11 but employing the dimethoxymethyl or formyl substituted proline ester of Col. I and the substituted benzeneamine shown in Col. II one obtains the L-proline ester product shown in Col. III which is then hydrogenated to the product shown in Col. IV.

Col. I

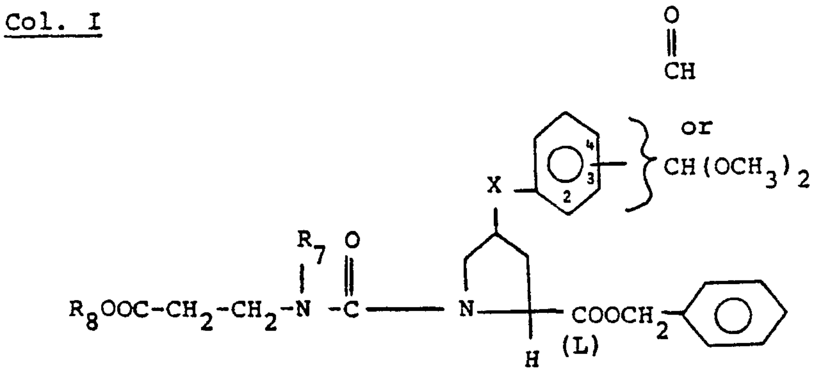

Col. II

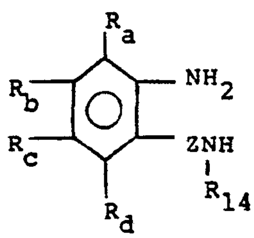

Col. III

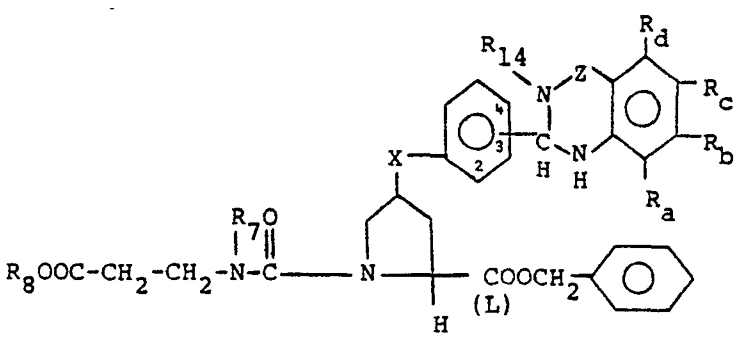

Col. IV

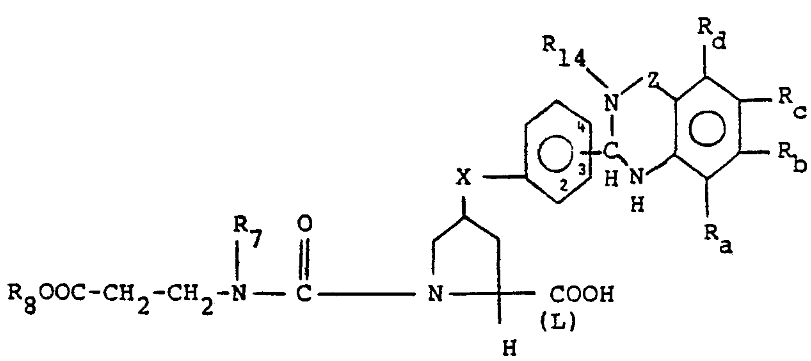

| Example | X | $R_8$ | $R_7$ | Positive | $R_a$ | $R_b$ | $R_c$ | $R_d$ | Z | $R_{14}$ |
|---|---|---|---|---|---|---|---|---|---|---|
| 29 | O | $H_5C_2-$ | $H_5C_2-$ | 2– | H | Cl | $SO_2NH_2$ | H | CO | $-CH_3$ |
| 30 | S | $H_5C_2-$ | $H_5C_2-$ | 4– | H | Cl | $SO_2NH_2$ | H | CO | H |
| 31 | O | $H_5C_2-$ | $H_5C_2-$ | 2– | H | Cl | $SO_2NH_2$ | H | $SO_2$ | H |
| 32 | S | $H_5C_2-$ | $H_5C_2-$ | 3– | H | Cl | $SO_2NH_2$ | H | $SO_2$ | $-CH_3$ |
| 33 | O | $H_5C_2-$ | $H_5C_2-$ | 4– | H | Cl | $SO_2NH_2$ | H | $SO_2$ | H |
| 34 | O | $H_5C_2-$ | $H_3C-$ | 2– | H | H | H | H | CO | H |
| 35 | S | $H_5C_2-$ | $(H_3C)_2-HC-$ | 3– | $CH_3$ | H | $SO_2NH_2$ | H | $SO_2$ | H |
| 36 | O | $H_5C_2-$ | $H_9C_4-$ | 4– | H | $OC_2H_5$ | Cl | H | CO | $-CH_2$ 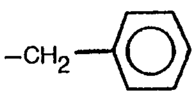 |
| 37 | O | $H_5C_2-$ | $H_5C_2-$ | 2– | H | H | $SO_2NH_2$ | Cl | $SO_2$ | H |
| 38 | O | $H_7C_3-$ | $H_5C_2-$ | 3– | H | Br | $SO_2NH_2$ | H | CO | H |
| 39 | O | $H_5C_2-$ | $H_5C_2-$ | 4– | H | CF | $SO_2NH_2$ | H | $SO_2$ | H |
| 40 | O | $H_3C-$ | $H_5C_2-$ | 3– | H | $CH_2CCl_3$ | $SO_2NH_2$ | H | CO | H |

The products of Col. IV of Examples 29 to 40 can be treated with aqueous sodium hydroxide according to the procedure of Example 12 to yield the corresponding products where $R_8$ is hydrogen.

Example 41

1000 tablets each containing the following ingredients:

| | | |
|---|---|---|
| [1(S),4S]-4-[3-[6-(Aminosulfonyl)-7-chloro-1,2,3,4-tetrahydro-4-oxo-2-quinazolinyl]phenoxy]-1-(3-mercapto-2-methyl-1-oxopropyl)-L-proline | 100 | mg. |
| Corn starch | 50 | mg. |
| Gelatin | 7.5 | mg. |
| Avicel® (microcrystalline cellulose) | 25 | mg. |
| Magnesium stearate | 2.5 | mg. |
| | 185 | mg. |

are prepared from sufficient bulk quantities by mixing the [1(S),4S] - 4 - [3 - [6 - (aminosulfonyl) - 7 - chloro - 1,2,3,4 - tetrahydro - 4 - oxo - 2 - quinazolinyl]phenoxy] - 1 - (3 - mercapto - 2 - methyl - 1 - oxopropyl) - L - proline and corn starch with an aqueous solution of the gelatin. The mixture is dried and ground to a fine powder. The Avicel® and then the magnesium stearate are admixed with granulation. This mixture is then compressed in a tablet press to form 1000 tablets each containing 100 mg of active ingredient.

In a similar manner, tablets containing 100 mg of the product of any of Examples 2 to 40 can be prepared.

Example 42

Two-piece #1 gelatin capsules each containing 100 mg of (cis) - 4 - [3 - [6 - (aminosulfonyl) - 7 - chloro - 1,2,3,4 - tetrahydro - 4 - oxo - 2 - quinazolinyl] - phenoxy - 1 - [[(3 - hydroxy - 3 - oxopropyl)ethylamino] - carbonyl] - L - proline are filled with a mixture of the following ingredients:

(cis-4-[3-[6-(Aminosulfonyl)-7-chloro-1,2,3,4-

| | | |
|---|---|---|
| tetrahydro-4-oxo-2-quinozolinyl]phenoxy]-1-[[(3-hydroxy-3-oxopropyl)ethylamino]carbonyl-L-proline | 100 | mg. |
| Magnesium stearate | 7 | mg. |
| Lactose | 193 | mg. |
| | 300 | mg. |

In a similar manner, capsules containing 100 mg of the product of any of Examples 1 to 11 and 13 to 40 can be prepared.

Example 43

An injectable solution is prepared as follows:

| | | |
|---|---|---|
| [1(S),4S]-4-[4-[6-(Aminosulfonyl)-7-chloro-1,2,3,4-tetrahydro-4-oxo-2-quinazolinyl]phenoxy]-1-[3-(benzoylthio)-2-methyl-1-oxopropyl]-L-proline | 500 | g. |
| Methyl paraben | 5 | g. |
| Propylparaben | 1 | g |
| Sodium chloride | 25 | g. |
| water for injection | 5 | l. |

The active substance, preservatives, and sodium chloride are dissolved in 3 liters of water for injection and then the volume is brought up to 5 liters. the solution is filtered through a sterile filter and asceptically filled into presterilized vials which are closed with presterilized rubber closures. Each vial contains 5 ml of solution in a concentration of 100 mg of active ingredient per ml of solution for injection.

In a similar manner, an injectable solution containing 100 mg of active ingredient per ml of solution can be prepared for the product of any of Examples 1 to 3 and 5 to 40.

## Claims

1. A compound of the formula

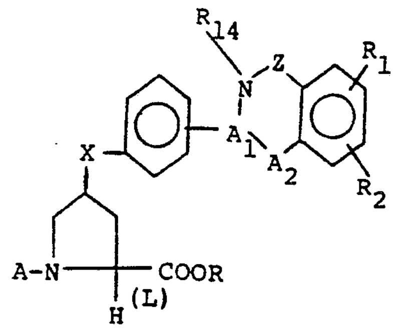

and a pharmaceutically acceptable salt thereof wherein

X is oxygen or sulfur;

$-A_1-A_2-$ is $-CH-NH-$ or $-C=N-$ .

A is 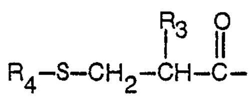 ,

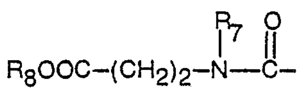 , 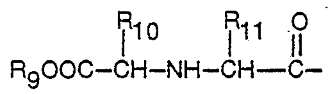 ,

or 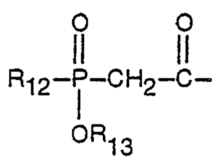 .

R, $R_8$ and $R_9$ are independently selected from hydrogen, $C_1$—$C_7$-alkyl, benzyl, benzhydryl, and salt forming ion;

$R_1$ and $R_2$ are independently selected from the group consisting of hydrogen, halogen $C_1$—$C_7$-alkyl, $C_1$—$C_7$-alkoxy, halo substituted $C_1$—$C_7$-alkyl, nitro, and $—SO_2NH_2$;

Z is 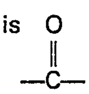 or 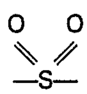 ;

$R_3$ is hydrogen, $C_1$—$C_7$-alkyl, halo substituted $C_1$—$C_7$-alkyl, phenyl, benzyl, phenethyl or $C_3$—$C_7$-cycloalkyl;

$R_4$ is hydrogen or

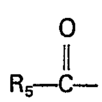 ;

$R_5$ is $C_1$—$C_7$-alkyl,

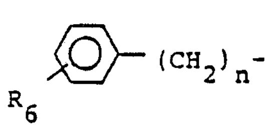 , 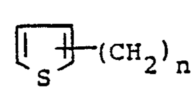 ,

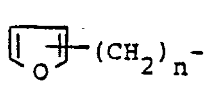 or 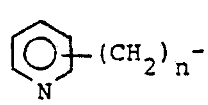 .

n is zero, one, two, three or four;
$R_6$ is hydrogen, $C_1$—$C_7$-alkyl, $C_1$—$C_7$-alkoxy, halogen or hydroxy;
$R_7$ is $C_1$—$C_7$-alkyl or $C_3$—$C_7$-cycloalkyl; $R_{10}$ is hydrogen, $C_1$—$C_7$-alkyl,

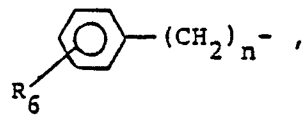 ,

halo substituted $C_1$—$C_7$-alkyl, hydroxy substituted $C_1$—$C_7$-alkyl, —$(CH_2)_q$-$C_3$—$C_7$-cycloalkyl, —$(CH_2)_q$-$N(C_1$—$C_7$-alkyl$)_2$, —$(CH_2)_q$-$NH_2$, —$(CH_2)_q$-carboxy, —$(CH_2)_q$-SH, —$(CH_2)_q$-S-$C_1$—$C_7$-alkyl,

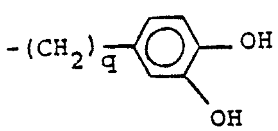 , 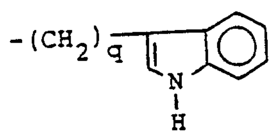 , 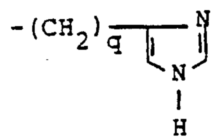 ,

$-(CH_2)_q$-guanidinyl, 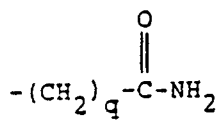 , 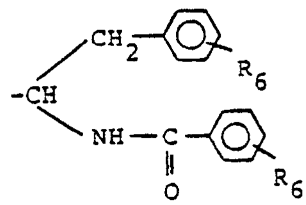 ;

q is one, two, three or four;
$R_{11}$ is hydrogen, $C_1$—$C_7$-alkyl, halo substituted $C_1$—$C_7$-alkyl, hydroxy substituted $C_1$—$C_7$-alkyl, —$(CH_2)_q$-$NH_2$, —$(CH_2)_q$-$N(C_1$—$C_7$-alkyl$)_2$,

$-(CH_2)_q$-guanidinyl, 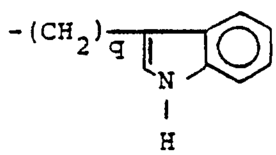 , 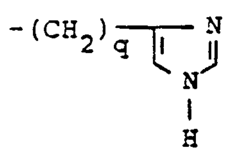 ,

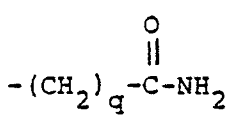 , $-CH_2-S-(CH_2)_2-NH_2$ ,

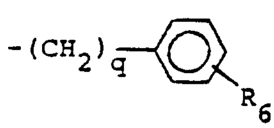 , 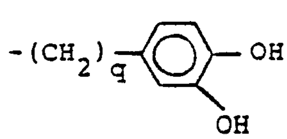 ,

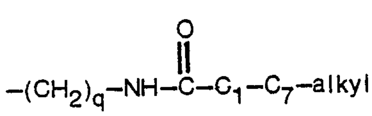 or 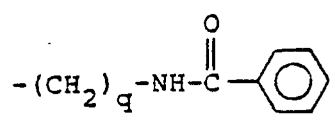 ;

$R_{12}$ is $C_1$—$C_{10}$-alkyl, 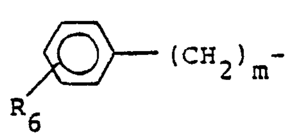 , 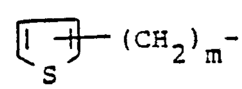 ,

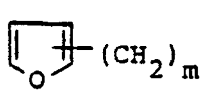 , 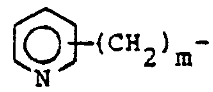 or $C_3$—$C_7$—cycloalkyl—$(CH_2)_m$— ;

m is zero or an integer from 1 to 7;

$R_{13}$ is hydrogen, $C_1$—$C_7$-alkyl, benzyl, benzhydryl, salt forming ion or

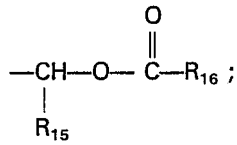

$R_{15}$ is hydrogen, $C_1$—$C_7$-alkyl, $C_3$—$C_7$-cycloalkyl or phenyl;
$R_{16}$ is hydrogen, $C_1$—$C_7$-alkyl, $C_1$—$C_7$-alkoxy, $C_3$—$C_7$-cycloalkyl, phenyl, benzyl, or phenethyl; and
$R_{14}$ is hydrogen, $C_1$—$C_7$-alkyl, $C_3$—$C_7$-cycloalkyl-$(CH_2)_n$—,

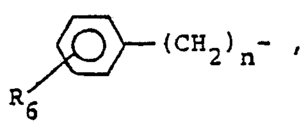

halo substituted $C_1$—$C_7$-alkyl, hydroxy substituted $C_1$—$C_7$-alkyl, —$(CH_2)_q$-$N(C_1$—$C_7$-$alkyl)_2$, or —$(CH_2)_q$-$NH_2$.

2. A compound of Claim 1 of the formula

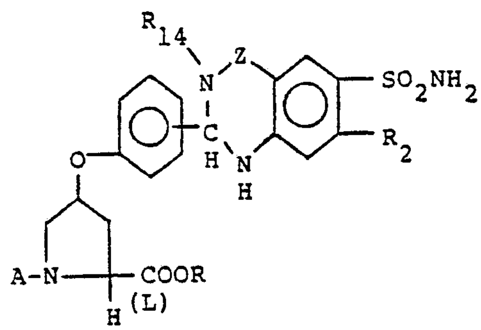

and a pharmaceutically acceptable salt thereof wherein
R is hydrogen, $C_1$—$C_4$-alkyl, or an alkali metal salt ion;
$R_{14}$ is hydrogen or $C_1$—$C_4$-alkyl;
$R_2$ is halogen, $C_1$—$C_4$-alkyl, or halo substituted $C_1$—$C_4$-alkyl;
$R_4$ is hydrogen or

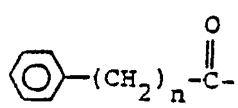

wherein n is zero, one or two;
$R_3$ is $C_1$—$C_4$-alkyl;
$R_8$ is hydrogen, $C_1$—$C_4$-alkyl, or an alkali metal salt ion;
$R_7$ is $C_1$—$C_4$-alkyl;
$R_9$ is hydrogen, $C_1$—$C_4$-alkyl, or an alkali metal salt ion;
$R_{10}$ is

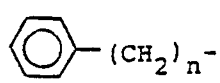

wherein n is zero, one, two, three or four;
$R_{11}$ is $C_1$—$C_4$-alkyl, —$CH_2$—S—$(CH_2)_2$—$NH_2$, or —$(CH_2)_4$-$NH_2$;
$R_{12}$ is $C_1$—$C_{10}$-alkyl

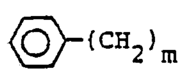

wherein m is zero or an integer from 1 to 7;

$R_{13}$ is hydrogen, alkali metal salt ion or

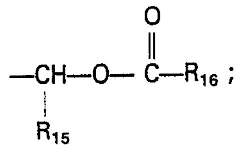

$R_{15}$ is hydrogen, straight or branched chain $C_1$—$C_4$-alkyl, or cyclohexyl; and
$R_{16}$ is straight or branched chain $C_1$—$C_4$-alkyl.

3. A compound of Claim 2 wherein
A is

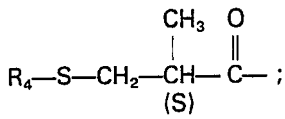

$R_{14}$ is hydrogen or methyl;
$R_2$ is chloro or trifluoromethyl; and
$R_4$ is hydrogen or

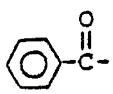

4. A compound of Claim 3 wherein

Z is 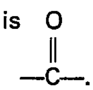

5. The compound of Claim 4, [1(S),4S]-4-[3-[6-(aminosulfonyl)-7-chloro-1,2,3,4-tetrahydro-4-oxo-2-quinazolinyl]phenoxy]-1-(3-mercapto-2-methyl-1-oxopropyl)-L-proline.

6. The compound of Claim 4, [1(S),4S]-4-[2-[6-(aminosulfonyl)-7-chloro-1,2,3,4-tetrahydro-4-oxo-2-quinazolinyl]phenoxy]-1-(3-mercapto-2-methyl-1-oxopropyl)-L-proline.

7. The compound of Claim 4, [1(S),4S]-4-[4-[6-(aminosulfonyl)-7-chloro-1,2,3,4-tetrahydro-4-oxo-2-quinazolinyl]phenoxy]-1-(3-mercapto-2-methyl-1-oxopropyl)-L-proline.

8. The compound of Claim 4, [1(S),4S]-4-[4-[6-(aminosulfonyl)-7-chloro-1,2,3,4-tetrahydro-4-oxo-2-quinazolinyl]phenoxy]-1-(3-benzoylthio-2-methyl-1-oxopropyl)-L-proline.

9. The compound of Claim 4, [1(S),4S]-4-[3-[6-(aminosulfonyl)-7-chloro-1,2,3,4-tetrahydro-4-oxo-2-quinazolinyl]phenoxy]-1-(3-benzoylthio-2-methyl-1-oxopropyl)-L-proline.

10. The compound of Claim 4, [1(S),4S]-4-[2-[6-(aminosulfonyl)-7-chloro-1,2,3,4-tetrahydro-4-oxo-2-quinazolinyl]phenoxy]-1-[3-(benzoylthio)-2-methyl-1-oxopropyl]-L-proline.

11. A compound of Claim 3 wherein Z is

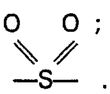

12. The compound of Claim 11, [1(S),4S]-4-[4-[7-(aminosulfonyl)-6-chloro-3,4-dihydro-1,1-dioxo-2H-1,2,4-benzothiadiazin-3-yl]phenoxy]-1-(3-mercapto-2-methyl-1-oxopropyl)-L-proline.

13. The compound of Claim 11, [1(S),4S]-4-[3-[7-(aminosulfonyl)-6-chloro-3,4-dihydro-1,1-dioxo-2H-1,2,4-benzothiadiazin-3-yl]phenoxy]-1-(3-mercapto-2-methyl-1-oxopropyl)-L-proline.

14. The compound of Claim 11, [1(S),4S]-4-[3-[7-(aminosulfonyl)-6-chloro-3,4-dihydro-1,1-dioxo-2H-1,2,4-benzothiadiazin-3-yl]phenoxy]-1-[3-(benzoylthio)-2-methyl-1-oxopropyl)-L-proline.

15. The compound of Claim 11, [1(S),4S]-4-[4-[7-(aminosulfonyl)-6-chloro-3,4-dihydro-1,1-dioxo-2H-1,2,4-benzothiadiazin-3-yl]phenoxy]-1-[3-benzoylthio)-2-methyl-1-oxopropyl]-L-proline.

16. The compound of Claim 11, [1(S),4S]-4-[4-[7-(aminosulfonyl)-3,4-dihydro-1,1-dioxo-6-(trifluoromethyl)-2H-1,2,4-benzothiadiazin-3-yl]phenoxy]-1-[3-(benzoylthio)-2-methyl-1-oxopropyl]-L-proline.

17. The compound of Claim 11, [1(S),4S]-4-[3-[7-(aminosulfonyl)-3,4-dihydro-1,1-dioxo-6-(trifluoromethyl)-2H-1,2,4-benzothiadiazin-3-yl]phenoxy]-1-[3-(benzoylthio)-2-methyl-1-oxopropyl)-L-proline.

18. The compound of Claim 11, [1(S),4S]-4-[4-[7-(aminosulfonyl)-6-chloro-3,4-dihydro-methyl-1,1-dioxo-2H-1,2,4-benzothiadiazin-3-yl]phenoxy]-1-[3-(benzothio)--2-methyl-1-oxopropyl)-L-proline.

19. A compound of Claim 2 wherein

A is

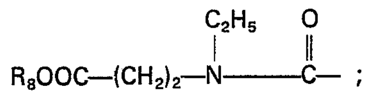

$R_{14}$ is hydrogen or methyl;

$R_2$ is chloro or trifluoromethyl; and

$R_8$ is hydrogen, ethyl, or an alkali metal salt ion.

20. The compound of Claim 19, (cis)-4-[3-[6-(aminosulfonyl)-7-chloro-1,2,3,4-tetrahydro-4-oxo-2-quinazolinyl]phenoxy]-1-[[(3-ethoxy-3-oxopropyl)ethylamino]carbonyl]-L-proline.

21. The compound of Claim 19, (cis)-4-[3-[6-(aminosulfonyl)-7-chloro-1,2,3,4-tetrahydro-4-oxo-2-quinazolinyl]phenoxy]-1-[[(3-hydroxy-3-oxopropyl)ethylamino]carbonyl]-L-proline.

22. A compound of Claim 2 wherein

A is

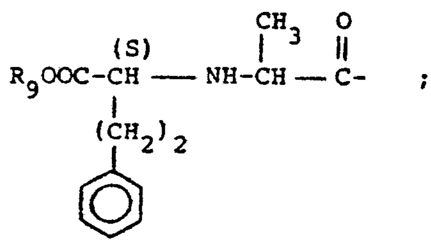

$R_{14}$ is hydrogen or methyl; and

$R_9$ is hydrogen, ethyl, or an alkali metal salt ion.

23. A compound of Claim 2 wherein

A is

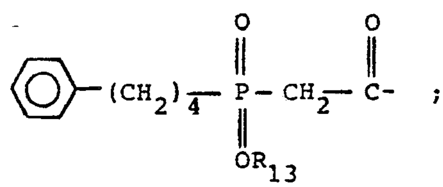

and

$R_{14}$ is hydrogen or methyl.

24. A composition useful for treating hypertension comprising a pharmaceutically acceptable carrier and a hypotensive agent or pharmaceutically acceptable salt thereof of the formula

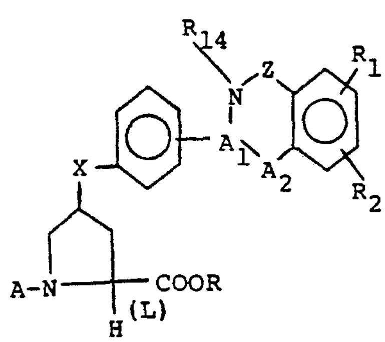

wherein A, $-A_1-A_2-$, R, $R_1$, $R_2$, $R_{14}$, X and Z are as defined in Claim 1.

**Patentansprüche**

1. Eine Verbindung der Formel

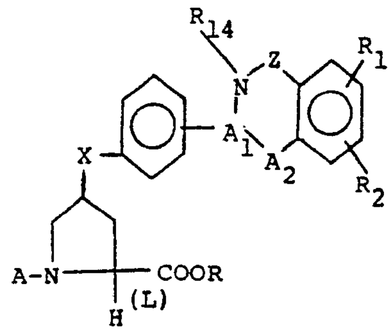

und ein pharmazeutisch verträgliches Salz davon, in der

X eine Sauerstoff- oder Schwefelatom bedeutet;

$-A_1-A_2-$ eine der Gruppen $-CH-NH-$ oder $-C=N-$ darstellt;

A einen der Reste

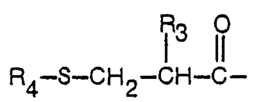 ,

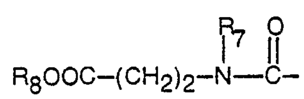 , 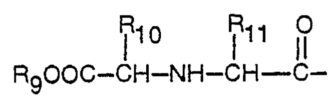 ,

oder 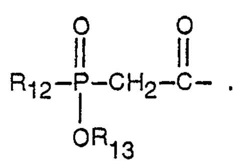 .

bedeutet;

R, $R_8$ und $R_9$ unabhängig voneinander Wasserstoffatome, $C_1$—$C_7$-Alkylrest, Benzyl-, oder Benzhydrylgruppen oder salzbildende Ionen darstellen;

$R_1$ und $R_2$ unabhängig voneinander Wasserstoff- oder Halogenatome, $C_1$—$C_7$-Alkyl-, $C_1$—$C_7$-Alkoxy- oder Halogen-substituierte $C_1$—$C_7$-Alkylreste, Nitrogruppen oder $—SO_2NH_2$ bedeuten;

Z eine der Gruppen

Z is 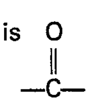 oder 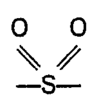 ;

$R_3$ ein Wasserstoffatom, einen $C_1$—$C_7$-Alkyl- oder einen Halogen-substituierten $C_1$—$C_7$-Alkylrest, eine Phenyl-, Benzyl- oder Phenäthylgruppe oder einen $C_3$—$C_7$-Cycloalkylrest bedeutet;

$R_4$ ein Wasserstoffatom oder den Rest

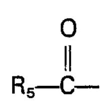 darstellt;

$R_5$ einen $C_1$—$C_7$-Alkylrest oder einen der Reste

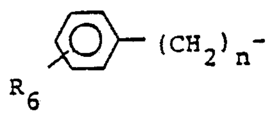 , 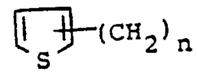 ,

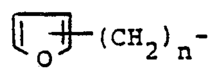 oder 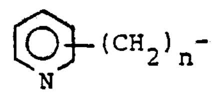 .

bedeutet;

n den Wert 0, 1, 2, 3 oder 4 hat;

$R_6$ ein Wasserstoffatom, einen $C_1$—$C_7$-Alkyl- oder $C_1$—$C_7$-Alkoxyrest, ein Halogenatom oder eine Hydroxylgruppe darstellt;

$R_7$ einen $C_1$—$C_7$-Alkyl- oder $C_3$—$C_7$-Cycloalkylrest bedeutet;

$R_{10}$ ein Wasserstoffatom, einen $C_1$—$C_7$-Alkylrest,

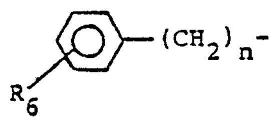 ,

einen Halogen-substituierten $C_1$—$C_7$-Alkylrest, Hydroxy-substituierten $C_1$—$C_7$-Alkylrest, einen der Reste —$(CH_2)_q$-$C_3$—$C_7$-cycloalkyl, —$(CH_2)_q$—$N(C_1$—$C_7$-$Alkyl)_2$, —$(CH_2)_q$—$NH_2$, —$(CH_2)_q$-Carboxy, —$(CH_2)_q$—SH, —$(CH_2)_q$—S—$C_1$—$C_7$-Alkyl,

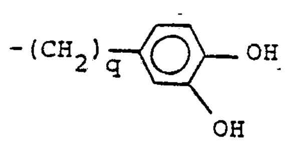 , 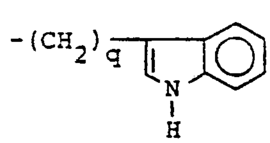 ,

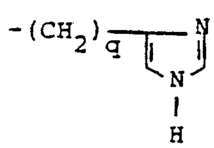 , $-(CH_2)_q$-guanidinyl,

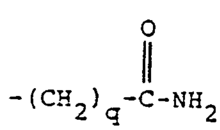 , 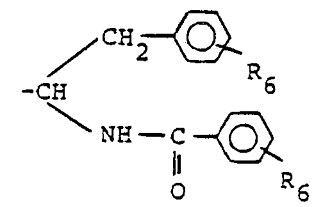 ;

q den Wert 1, 2, 3 oder 4 hat;

$R_{11}$ ein Wasserstoffatom, einen $C_1$—$C_7$-Alkyl-, Halogen-substituierten $C_1$—$C_7$-Alkyl- oder Hydroxy-substituierten $C_1$—$C_7$-Alkyl- rest oder einen der Reste —$(CH_2)_q$—$NH_2$, —$(CH_2)_q$—$N(C_1$—$C_7$-Alkyl$)_2$,

$-(CH_2)_q$-guanidinyl, 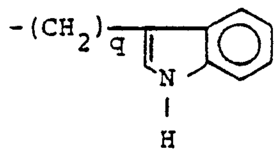 ,

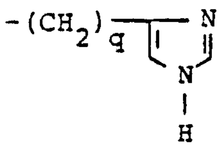 , 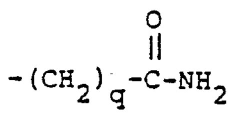 , $-CH_2-S-(CH_2)_2-NH_2$ ,

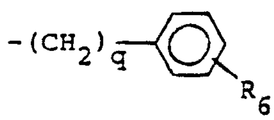 , 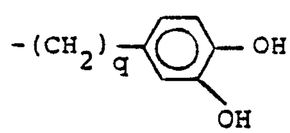 ,

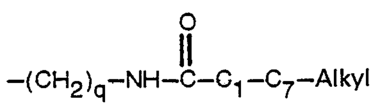 oder 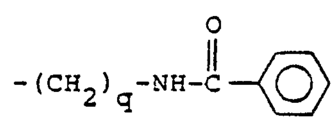 ;

darstellt;

$R_{12}$ einen $C_1$-$C_{10}$-Alkylrest oder einen Rest der Formel

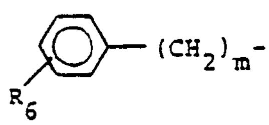 , 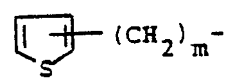 ,

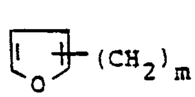 , 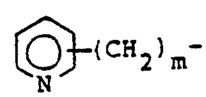 oder

$C_3$-$C_7$-Cycloalkyl-$(CH_2)_m$- bedeutet;

m den Wert 0 hat oder eine ganze Zahl im Wert von 1 bis 7 ist;

$R_{13}$ ein Wasserstoffatom, einen $C_1$—$C_7$-Alkylrest, eine Benzyl- oder Benzhydrylgruppe oder ein Salzbildendes Ion oder einen Rest der Formel

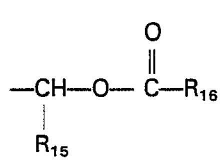 bedeutet;

$R_{15}$ ein Wasserstoffatom, einen $C_1$—$C_7$-Alkyl- oder $C_3$—$C_7$-Cycloalkylrest oder eine Phenylgruppe darstellt;

$R_{16}$ ein Wasserstoffatom, einen $C_1$—$C_7$-Alkyl-, $C_1$—$C_7$-Alkoxy- oder $C_3$—$C_7$-Cycloalkylrest, eine Phenyl-, Benzyl- oder Phenäthylgruppe darstellt; und

$R_{14}$ ein Wasserstoffatom, einen $C_1$—$C_7$-Alkylrest, einen Rest der Formel $C_3$—$C_7$-Cycloalkyl-$(CH_2)_n$— oder

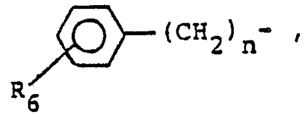

einen Halogen-substituierten $C_1$—$C_7$-Alkyl- oder Hydroxy-substituierten $C_1$—$C_7$-Alkylrest oder einen Rest der Formel $-(CH_2)_q-N(C_1-C_7\text{-Alkyl})_2$ oder $-(CH_2)_q-NH_2$ bedeutet.

2. Eine Verbindung nach Anspruch 1 der Formel

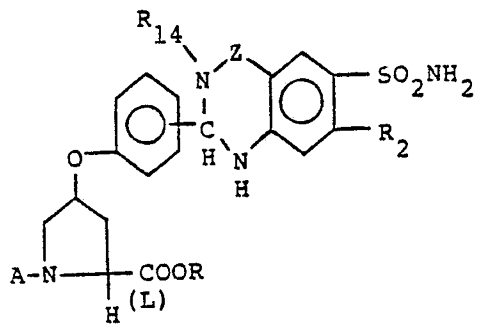

und ein pharmazeutisch verträgliches Salz davon, in der R eine Wasserstoffatom, einen $C_1$—$C_4$-Alkylrest oder ein Alkalimetallsalzion bedeutet;

$R_{14}$ ein Wasserstoffatom oder einen $C_1$—$C_4$-Alkylrest darstellt;

$R_2$ ein Halogenatom, einen $C_1$—$C_4$-Alkyl- oder einen Halogen-substituierten $C_1$—$C_4$-Alkylrest bedeutet;

$R_4$ ein Wasserstoffatom oder einen Rest der Formel

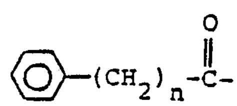

bedeutet, in der n den Wert 0, 1 oder 2 hat;

$R_3$ einen $C_1$—$C_4$-Alkylrest darstellt;

$R_8$ ein Wasserstoffatom, einen $C_1$—$C_4$-Alkylrest oder ein Alkalimetallsalzion darstellt;

$R_7$ einen $C_1$—$C_4$-Alkylrest bedeutet;

$R_9$ ein Wasserstoffatom, einen $C_1$—$C_4$-Alkylrest oder ein Alkalimetallsalzion darstellt;

$R_{10}$ einen Rest der Formel

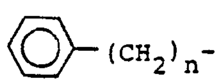

bedeutet, in dem n den Wert 0, 1, 2, 3 oder 4 hat;

$R_{11}$ einen $C_1$—$C_4$-Alkylrest oder eine der Gruppen $-CH_2-S-(CH_2)_2-NH_2$ oder $-CH_2)_4-NH_2$ darstellt;

$R_{12}$ einen $C_1$—$C_{10}$-Alkylrest oder einen Rest der Formel

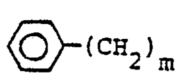

bedeutet, in der m den Wert 0 hat oder eine ganze Zahl im Wert von 1 bis 7 ist;

$R_{13}$ ein Wasserstoffatom, eine Alkalimetallsalzion oder einen Rest der Formel

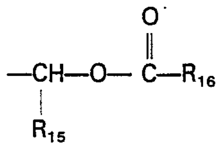

darstellt;

$R_{15}$ ein Wasserstoffatom, einen unverzweigten oder verzweigten $C_1$—$C_4$-Alkylrest oder eine Cyclohexylgruppe bedeutet; und

$R_{16}$ edinen unverzweigten oder verzweigten $C_1$—$C_4$-Alkylrest darstellt.

3. Eine Verbindung nach Anspruch 2, in der A einen Rest der Formel

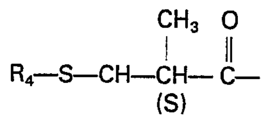

bedeutet;

$R_{14}$ ein Wasserstoffatom oder eine Methylgruppe darstellt;

$R_2$ ein Chloratom oder eine Trifluormethylgruppe bedeutet; und

$R_4$ ein Wasserstoffatom oder die Gruppe

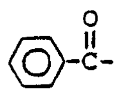

darstellt.

4. Eine Verbindung nach Anspruch 3, in der Z die Gruppe

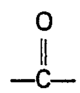

bedeutet.

5. Verbindung nach Anspruch 4, nämlich [1(S),4S]-4-[3-[6-(Aminosulfonyl)-7-chlor-1,2,3,4-tetrahydro-4-oxo-2-quinazolinyl]-phenoxy]-1-(3-mercapto-2-methyl-1-oxopropyl)-L-prolin.

6. Verbindung nach Anspruch 4, nämlich [1(S),4S]-4-[2-[6-(Aminosulfonyl)-7-chlor-1,2,3,4-tetrahydro-4-oxo-2-quinazolinyl]-phenoxy]-1-(3-mercapto-2-methyl-1-oxopropyl)-L-prolin.

7. Verbindung nach Anspruch 4, nämlich [1(S),4S]-4-[4-[6-(Aminosulfonyl)-7-chlor-1,2,3,4-tetrahydro-4-oxo-2-quinazolinyl]-phenoxy]-1-(3-mercapto-2-methyl-1-oxopropyl)-L-prolin.

8. Verbindung nach Anspruch 4, nämlich [1(S),4S]-4-[4-[6-(Aminosulfonyl)-7-chlor-1,2,3,4-tetrahydro-4-oxo-2-quinazolinyl]-phenoxy]-1-(3-benzoylthio)-2-methyl-1-oxopropyl)-L-prolin.

9. Verbindung nach Anspruch 4, nämlich [1(S),4S]-4-[3-[6-(Aminosulfonyl)-7-chlor-1,2,3,4-tetrahydro-4-oxo-2-quinazolinyl]-phenoxy]-1-(3-benzoylthio)-2-methyl-1-oxopropyl)-L-prolin.

10. Verbindung nach Anspruch 4, nämlich [1(S),4S]-4-[2-[6-(Aminosulfonyl)-7-chlor-1,2,3,4-tetrahydro-4-oxo-2-quinazolinyl]-phenoxy]-1-(3-benzoylthio)-2-methyl-1-oxopropyl)-L-prolin.

11. Eine Verbindung nach Anspruch 3, in der Z die Gruppe

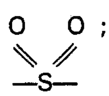

bedeutet.

12. Verbindung nach Anspruch 11, nämlich [1(S),4S]-4-[4-[7-(Aminosulfonyl)-6-chlor-3,4-dihydro-1,1-dioxo-2H-1,2,4-benzothiadiazin-3-yl]-phenoxy]-1-(3-mercapto-2-methyl-1-oxopropyl)-L-prolin.

13. Verbindung nach Anspruch 11, nämlich [1(S),4S]-4-[3-[7-(Aminosulfonyl)-6-chlor-3,4-dihydro-1,1-dioxo-2H-1,2,4-benzothiadiazin-3-yl]-phenoxy]-1-(3-mercapto-2-methyl-1-oxopropyl)-L-prolin.

14. Verbindung nach Anspruch 11, nämlich [1(S),4S]-4-[3-[7-(Aminosulfonyl)-6-chlor-3,4-dihydro-1,1-dioxo-2H-1,2,4-benzothiadiazin-3-yl]-phenoxy]-1-[3-(benzoylthio)-2-methyl-1-oxopropyl)-L-prolin.

15. Verbindung nach Anspruch 11, nämlich [1(S),4S]-4-[4-[7-(Aminosulfonyl)-6-chlor-3,4-dihydro-1,1-dioxo-2H-1,2,4-benzothiadiazin-3-yl]-phenoxy]-1-[3-benzoylthio)-2-methyl-1-oxopropyl)-L-prolin.

16. Verbindung nach Anspruch 11, nämlich [1(S),4S]-4-[4-[7-(Aminosulfonyl)-3,4-dihydro-1,1-dioxo-6-(trifluoromethyl)-2H-1,2,4-benzothiadiazin-3-yl]-phenoxy]-1-[3-benzoylthio)-2-methyl-1-oxopropyl)-L-prolin.

17. Verbindung nach Anspruch 11, nämlich [1(S),4S]-4-[3-[7-(Aminosulfonyl)-3,4-dihydro-1,1-dioxo-6-(trifluoromethyl)-2H-1,2,4-benzothiadiazin-3-yl]-phenoxy]-1-[(3-benzoylthio)-2-methyl-1-oxopropyl)-L-prolin.

18. Verbindung nach Anspruch 11, nämlich [1(S),4S]-4-[4-[7-(Aminosulfonyl)-6-chlor-3,4-dihydro-2-methyl-1,1-dioxo-2H-1,2,4-benzothiadiazin-3-yl]-phenoxy]-1-[(3-benzothio)-2-methyl-1-oxopropyl)-L-prolin.

19. Eine Verbindung nach Anspruch 2, in der A einen Rest der Formel

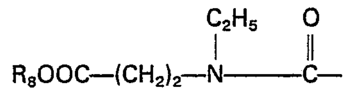 ;

bedeutet;

$R_{14}$ ein Wasserstoffatom oder eine Methylgruppe darstellt;

$R_2$ ein Chloratom oder eine Trifluormethylgruppe bedeutet; und

$R_8$ ein Wasserstoffatom, eine Äthylgruppe oder ein Alkalimetallsalzion darstellt.

20. Verbindung nach Anspruch 19, nämlich (cis)-4-[3-[6-(Aminosulfonyl)-7-chlor-1,2,3,4-tetrahydro-4-oxo-2-quinazolinyl]-phenoxy]-1-[[(3-äthoxy-3-oxopropyl)-äthylamino]-carbonyl]-L-prolin.

21. Verbindung nach Anspruch 19, nämlich (cis)-4-[3-[6-(Aminosulfonyl)-7-chlor-1,2,3,4-tetrahydro-4-oxo-2-quinazolinyl]-phenoxy]-1-[[(3-hydroxy-3-oxopropyl)-äthylamino]-carbonyl]-L-prolin.

22. Eine Verbindung nach Anspruch 2, in der A einen Rest der Formel

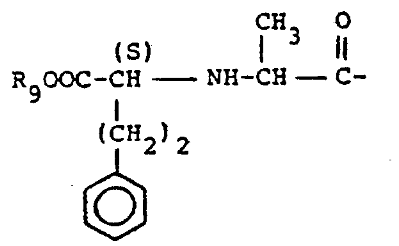 ;

bedeutet;

$R_{14}$ ein Wasserstoffatom oder eine Methylgruppe darstellt; und

$R_9$ eine Wasserstoffatom, eine Äthylgruppe oder ein Alkalimetallsalzion bedeutet.

23. Eine Verbindung nach Anspruch 2, in der A einen Rest der Formel

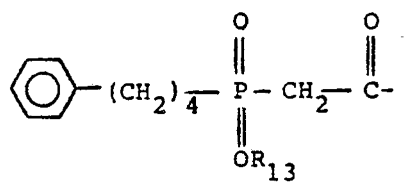 ;

bedeutet; und

$R_{14}$ ein Wasserstoffatom oder eine Methylgruppe darstellt.

24. Ein zur Behandlung von Bluthochdruck geeignetes Mittel, umfassend einen pharmazeutisch verträglichen Träger und einen blutdrucksenkenden Wirkstoff und ein pharmazeutisch verträgliches Salz davon der Formel

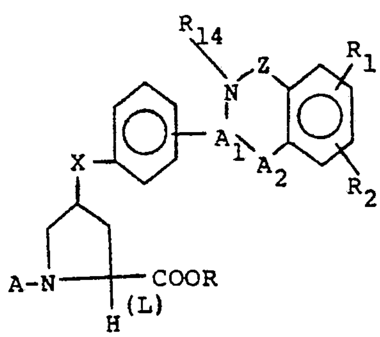

in der A, —$A_1$—$A_2$—, R, $R_1$, $R_2$, $R_{14}$, X und Z wie in Anspruch 1 definiert sind.

**Revendications**

1. Composé de formule

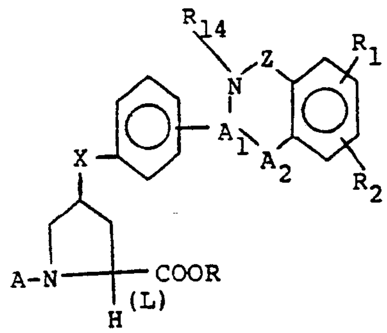

et sels pharmaceutiquement acceptables de celui-ci, formule dans laquelle

X est un atome d'oxygène ou de soufre;

$-A_1-A_2-$ est $-CH-NH-$ ou $-C=N-$.

A est 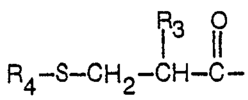 ,

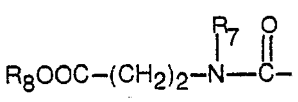 , 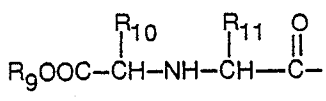 ,

ou 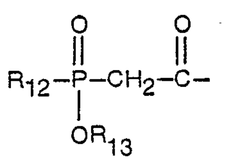 .

R, $R_8$ et $R_9$ sont choisis, indépendamment, entre les atomes d'hydrogène, les radicaux alkyle en $C_1$—$C_7$, benzyle et benzhydryle, et les ions formateurs de sels;

$R_1$ et $R_2$ sont choisis, indépendamment, entre les atomes d'hydrogène et d'halogène, les radicaux alkyle en $C_1$—$C_7$, alcoxy en $C_1$—$C_7$, alkyl en $C_1$—$C_7$ substitués par halogène, et les groupements nitro et $—SO_2NH_2$;

Z est

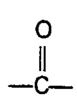 ou 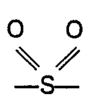 ;

$R_3$ est un atome d'hydrogène ou un radical alkyle en $C_1$—$C_7$, alkyle en $C_1$—$C_7$ substitué par halogène, phényle, benzyle, phénéthyle ou cycloalkyle en $C_3$—$C_7$;

$R_4$ est un atome d'hydrogène ou

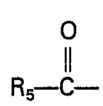 ;

$R_5$ est un radical alkyle en $C_1$—$C_7$,

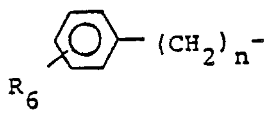 , 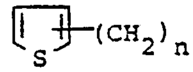 ,

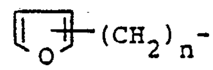 ou 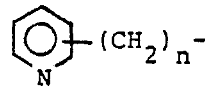 .

n est égal à zéro, un, deux, trois ou quatre;

$R_6$ est un atome d'hydrogène, un radical alkyle en $C_1$—$C_7$ ou alcoxy en $C_1$—$C_7$, un atome d'halogène, ou un groupement hydroxy;

$R_7$ est un radical alkyle en $C_1$—$C_7$ ou cycloalkyle en $C_3$—$C_7$;

$R_{10}$ est un atome d'hydrogène ou un radical alkyle en $C_1$—$C_7$,

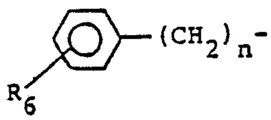 ,

alkyle en $C_1$—$C_7$ substitué par halogène, alkyle en $C_1$—$C_7$ substitué par hydroxy, —$(CH_2)_q$-cycloalkyle en $C_3$—$C_7$, —$(CH_2)_q$—N(alkyle en $C_1$—$C_7)_2$, —$(CH_2)_q$—$NH_2$, —$(CH_2)_q$-carboxy, —$(CH_2)_q$—SH, —$(CH_2)_q$—S—alkyle en $C_1$—$C_7$,

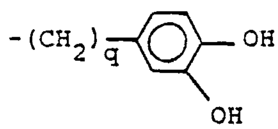 , 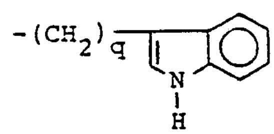 ,

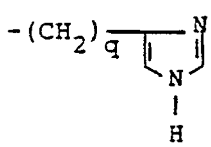 , $-(CH_2)_q-$guanidinyle,

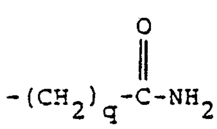 ou 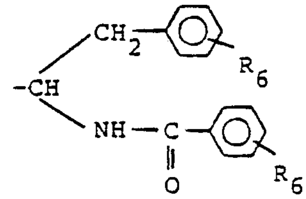 ;

q est égal à un, deux, trois ou quatre;

$R_{11}$ est un atome d'hydrogène ou un radical alkyle en $C_1$—$C_7$, alkyle en $C_1$—$C_7$ substitué par halogène, alkyle en $C_1$—$C_7$ substitué par hydroxy, —$(CH_2)_q$—$NH_2$, —$(CH_2)_q$—N (alkyle en $C_1$—$C_7)_2$,

$-(CH_2)_q$-guanidinyle, 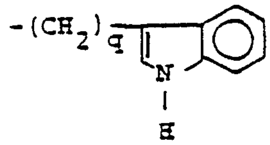 ,

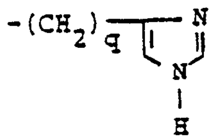 , $-(CH_2)_q-\overset{O}{\overset{\|}{C}}-NH_2$ , $-CH_2-S-(CH_2)_2-NH_2$ ,

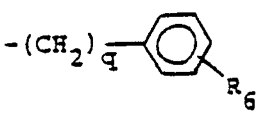 , 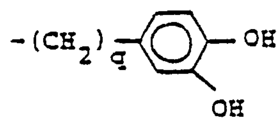 ,

$-(CH_2)_q-NH-\overset{O}{\overset{\|}{C}}-$alkyle en $C_1-C_7$ ou 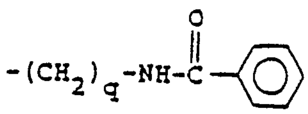 ;

$R_{12}$ est un radical alkyle en $C_1-C_{10}$,

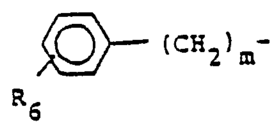 , 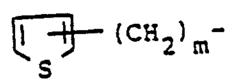 ,

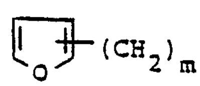 , 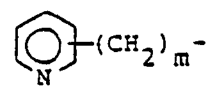 ou

cycloalkyl en $C_3C_7-(CH_2)_m-$ ;

m est égal à zero ou est un nombre entier de 1 à 7;

$R_{13}$ est un atome d'hydrogène, un radical alkyle en $C_1—C_7$, benzyle ou benzhydryle, un ion formateur de sel ou un groupement de formule

$$-\underset{\substack{|\\R_{15}}}{CH}-O-\overset{O}{\overset{\|}{C}}-R_{16}\ ;$$

$R_{15}$ est un atome d'hydrogène ou un radical alkyle en $C_1—C_7$, cycloalkyle en $C_3—C_7$ ou phényle;

$R_{16}$ est un atome d'hydrogène ou un radical alkyle en $C_1—C_7$, alcoxy en $C_1—C_7$, cycloalkyle en $C_3—C_7$, phényle, benzyle ou pohénéthyle; et

$R_{14}$ est un atome d'hydrogène ou un radical alkyle en $C_1—C_7$, cycloalkyl en $C_3—C_7—(CH_2)_n—$,

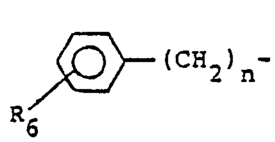 ,

alkyle en $C_1—C_7$ substitué par halogène, alkyle en $C_1—C_7$ substitué par hydroxy, $—(CH_2)_q—N$(alkyle en $C_1—C_7)_2$, ou $—(CH_2)_q—NH_2$.

2. Composé selon la revendication 1, de formule

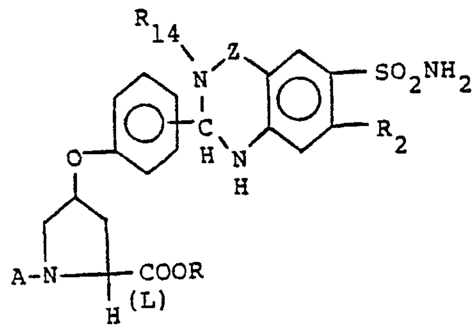

et sel pharmaceutiquement acceptable de celui-ci, formule dans laquelle

R est un atome d'hydrogène, un radical alkyle en $C_1$—$C_4$, ou un ion sel de métal alcalin;

$R_{14}$ est un atome d'hydrogène ou un radical alkyle en $C_1$—$C_4$;

$R_2$ est un atome d'halogène ou un radical alkyle en $C_1$—$C_4$ éventuellement substitué par halogéne;

$R_4$ est un atome d'hydrogène ou

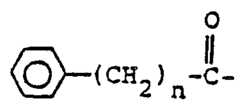

où n est égal à zéro, un ou deux;

$R_3$ est un radical alkyle en $C_1$—$C_4$;

$R_8$ est un atome d'hydrogène, un radical alkyle en $C_1$—$C_4$ ou un ion sel de métal alcalin;

$R_7$ est un radical alkyle en $C_1$—$C_4$;

$R_9$ est un atome d'hydrogène, un radical alkyle en $C_1$—$C_4$ ou un ion sel de métal alcalin;

$R_{10}$ est

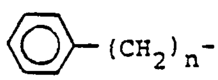

où n est égal à zéro, un, deux, trois ou quatre;

$R_{11}$ est un radical alkyle en $C_1$—$C_4$ ou un groupement de formule —$CH_2$—S—$(CH_2)_2$—$NH_2$, ou —$(CH_2)_4$—$NH_2$;

$R_{12}$ est un radical alkyle en $C_1$—$C_{10}$ ou

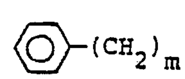

où m est égal à zéro ou est un nombre entier de 1 à 7;

$R_{13}$ est un atome d'hydrogène, un ion sel de métal alcalin ou

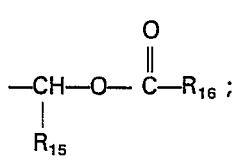

$R_{15}$ est un atome d'hydrogène, un radical alkyle en $C_1$—$C_4$ à chaîne droite ou ramifiée, ou un radical cyclohexyle; et

$R_{16}$ est un radical alkyle en $C_1$—$C_4$ à chaîne droite ou ramifiée.

3. Composé selon la revendication 2, dans la formule duquel

A est 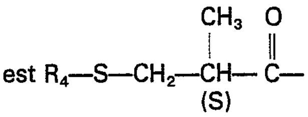 ;

$R_{14}$ est un atome d'hydrogène ou un radical méthyle;
$R_2$ est un atome de chlore ou un radical trifluorométhyle; et
$R_4$ est un atome d'hydrogène ou

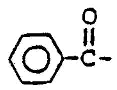

4. Composé selon la revendication 3, dans la formule duquel Z est

Z is 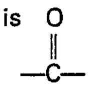

5. Composé selon la revendication 4, qui est la [1(S),4S]-4-[3-[6-(aminosulfonyl)-7-chloro-1,2,3,4-tétrahydro-4-oxo-2-quinazolinyl]phénoxy]-1-(3-mercapto-2-méthyl-1-oxopropyl)-L-proline.

6. Composé selon la revendication 4, qui est la [1(S),4S]-4-[2-[6-(aminosulfonyl)-7-chloro-1,2,3,4-tétrahydro-4-oxo-2-quinazolinyl]phénoxy]-1-(3-mercapto-2-méthyl-1-oxopropyl)-L-proline.

7. Composé selon la revendication 4, qui est la [1(S),4S]-4-[4-[6-(aminosulfonyl)-7-chloro-1,2,3,4-tétrahydro-4-oxo-2-quinazolinyl]phénoxy]-1-(3-mercapto-2-méthyl-1-oxopropyl)-L-proline.

8. Composé selon la revendication 4, qui est la [1(S),4S]-4-[4-[6-(aminosulfonyl)-7-chloro-1,2,3,4-tétrahydro-4-oxo-2-quinazolinyl]phénoxy]-1-(3-benzoylthio)-2-méthyl-1-oxopropyl)-L-proline.

9. Composé selon la revendication 4, qui est la [1(S),4S]-4-[3-[6-(aminosulfonyl)-7-chloro-1,2,3,4-tétrahydro-4-oxo-2-quinazolinyl]phénoxy]-1-(3-benzoylthio)-2-méthyl-1-oxopropyl)-L-proline.

10. Composé selon la revendication 4, qui est la [1(S),4S]-4-[2-[6-(aminosulfonyl)-7-chloro-1,2,3,4-tétrahydro-4-oxo-2-quinazolinyl]phénoxy]-1-[3-(benzoylthio)-2-méthyl-1-oxopropyl]-L-proline.

11. Composé selon la revendication 3, dans la formule duquel
Z est

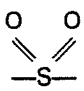 ;

12. Composé selon la revendication 11, qui est la [1(S),4S]-4-[4-[7-(aminosulfonyl)-6-chloro-3,4-dihydro-1,1-dioxo-2H-1,2,4-benzothiadiazin-3-yl]phénoxy]-1-(3-mercapto-2-méthyl-1-oxopropyl)-L-proline.

13. Composé selon la revendication 11, qui est la [1(S),4S]-4-[3-[7-(aminosulfonyl)-6-chloro-3,4-dihydro-1,1-dioxo-2H-1,2,4-benzothiadiazin-3-yl]phénoxy]-1-(3-mercapto-2-méthyl-1-oxopropyl)-L-proline.

14. Composé selon la revendication 11, qui est la [1(S),4S]-4-[3-[7-(aminosulfonyl)-6-chloro-3,4-dihydro-1,1-dioxo-2H-1,2,4-benzothiadiazin-3-yl]phénoxy]-1-[3-(benzoylthio)-2-méthyl-1-oxopropyl)-L-proline.

15. Composé selon la revendication 11, qui est la [1(S),4S]-4-[4-[7-(aminosulfonyl)-6-chloro-3,4-dihydro-1,1-dioxo-2H-1,2,4-benzothiadiazin-3-yl]phénoxy]-1-(3-benzoylthio-2-méthyl-1-oxopropyl)-L-proline.

16. Composé selon la revendication 11, qui est la [1(S),4S]-4-[4-[7-(aminosulfonyl)-3,4-dihydro-1,1-dioxo-6-(trifluoromethyl)-2H-1,2,4-benzothiadiazin-3-yl]phénoxy]-1-(3-benzoylthio-2-méthyl-1-oxopropyl)-L-proline.

17. Composé selon la revendication 11, qui est la [1(S),4S]-4-[3-[7-(aminosulfonyl)-3,4-dihydro-1,1-dioxo-6-(trifluoromethyl)-2H-1,2,4-benzothiadiazin-3-yl]phénoxy]-1-(3-benzoylthio-2-méthyl-1-oxopropyl)-L-proline.

18. Composé selon la revendication 11, qui est la [1(S),4S]-4-[4-[7-(aminosulfonyl)-6-chloro-3,4-dihydro-2-méthyl-1,1-dioxo-2H-1,2,4-benzothiadiazin-3-yl]phénoxy]-1-(3-benzoylthio-2-méthyl-1-oxopropyl)-L-proline.

19. Composé selon la revendication 2, dans la formule duquel

A est 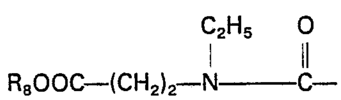 ;

$R_{14}$ est un atome d'hydrogène ou un radical méthyle;

$R_2$ est un atome de chlore ou un radical trifluorométhyle; et

$R_8$ est un atome d'hydrogène, un radical éthyle ou un ion sel de métal alcalin.

20. Composé selon la revendication 19, qui est la (cis)-4-[3-[6-(aminosulfonyl)-7-chloro-1,2,3,4-tétrahydro-4-oxo-2-quinazolinyl]phénoxy]-1-[[(3-éthoxy-3-oxopropyl)éthylamino]carbonyl]-L-proline.

21. Composé selon la revendication 19, qui est la (cis)-4-[3-[6-(aminosulfonyl)-7-chloro-1,2,3,4-tétrahydro-4-oxo-2-quinazolinyl]phénoxy]-1-[[(3-hydroxy-3-oxopropyl)éthylamino]carbonyl]-L-proline.

22. Composé selon la revendication 2, dans la formule duquel

A est

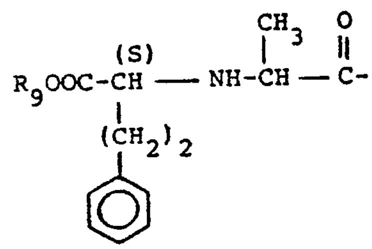

;

$R_{14}$ est un atome d'hydrogène ou un radical méthyle; et

$R_9$ est un atome d'hydrogène, un radical éthyle ou un ion sel de métal alcalin.

23. Composé selon la revendication 2, dans la formule duquel

A est

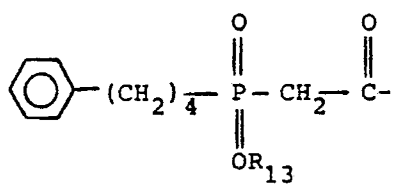

;

et

$R_{14}$ est un atome d'hydrogène ou un radical méthyle.

24. Composition utilisable pour le traitement de l'hypertension, comprenant un porteur pharmaceutiquement acceptable et un agent antihypertenseur ou un sel pharmaceutiquement acceptable de celui-ci, de formule

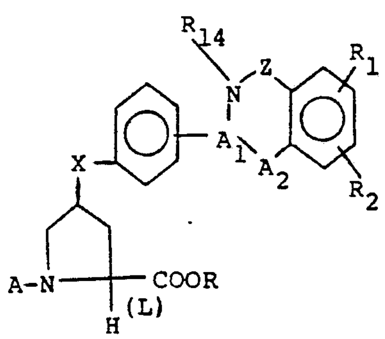

dans laquelle A, —$A_1$—$A_2$—, R, $R_1$, $R_2$, $R_{14}$, X et Z sont tels que définis dans la revendication 1.